# EUROPEAN PATENT SPECIFICATION

(11) **EP 2 575 580 B1**
(45) Date of publication and mention of the grant of the patent: **16.11.2016**
(21) Application number: 11789335.4
(22) Date of filing: 03.05.2011
(51) Int. Cl.: A47K 7/00, D21H 19/10, D21H 21/14

(54) **SINGLE-PLY DISPERSIBLE WET WIPES WITH ENHANCED DISPERSIBILITY**
DISPERGIERBARE FEUCHTE EINZELSCHICHT-WISCHTÜCHER MIT VERSTÄRKTER DISPERGIERBARKEIT
SERVIETTES HUMIDES DISPERSIBLES À PLI UNIQUE PRÉSENTANT UNE DISPERSIBILITÉ AMÉLIORÉE

(30) Priority: 01.06.2010 US 791521
(43) Date of publication of application: 10.04.2013
(73) Proprietor: Kimberly-Clark Worldwide, Inc., Neenah, Wisconsin 54956 (US)
(72) Inventor: VOGEL, Nathan, John, Neenah Wisconsin 54956 (US); ZWICK, Kenneth, John, Neenah Wisconsin 54956 (US); POWLING, David, James Sealy, Combined Locks Wisconsin 54113 (US); JOHNSON, Kroy, Donald, Neenah Wisconsin 54956 (US); LORTSCHER, Peter, Shawn, Neenah Wisconsin 54956 (US); MONSON, Robert, Stanley, Neenah Wisconsin 54956 (US); BIGGS, David, Glen, Neenah Wisconsin 54956 (US); SHLEPR, Michael, George, Greenville Wisconsin 54952 (US)
(74) Representative: Davies, Christopher Robert
(86) International application number: PCT/IB2011/051956
(87) International publication number: WO 2011/151749

(56) References cited:
- WO-A1-2006/011932
- WO-A1-2008/132614
- JP-A- 2002 325 698
- JP-A- 2002 506 138
- JP-A- 2004 016 637
- JP-A- 2006 512 514
- US-A1- 2004 058 606

## Description

### BACKGROUND

Dispersible flushable moist products must exhibit satisfactory in-use strength, but quickly break down in sewer or septic systems. Current flushable moist wipes do this by using a triggerable salt sensitive binder on a substrate comprising cellulose based fibers. The binder attaches to cellulose fibers which form a network of in-use strength in a 2 percent salt solution (used as the moist wipe formulation), but swells and falls apart in the fresh water of the toilet and sewer system.

Additionally, flushable moist wipes need to easily pass through current municipal sewer systems. For many years, the problem of disposability has plagued industries that provide disposable items, such as diapers, wet wipes, incontinence garments and feminine care products. Ideally, when a flushable disposable product is discarded in either sewer or septic systems, the product, or designated portions of the product, should "disperse" and thus sufficiently dissolve or disintegrate in water so as not to present problems under conditions typically found in household and municipal sanitization systems. Some products have failed to properly disperse. Many current wipe manufacturers achieve acceptable strength in flushable moist wipes by using long fibers (>10 mm) which entangle with other fibers to develop a wet strength network. However, these long fibers are not desirable because they tend to collect on screens in waste water systems and cause obstructions and blockages.

As a result, there has been a movement by municipalities to define "flushable" through various regulations. Flushable moist wipes must meet these regulations to allow for compatibility with home plumbing fixtures and drain lines, as well as the disposal of the product in onsite and municipal wastewater treatment systems. By following these regulations, manufacturers can ensure that in normal conditions products best disposed of via the waste water systems for public health and hygiene reasons will not block toilets, drainage pipes, water conveyance and treatments systems or become an aesthetic nuisance in surface waters or soil environments.

One challenge for flushable moist wipes is that they take much longer to break down when compared to dry toilet tissue potentially creating issues in sewer or septic systems. Currently dry toilet tissue quickly exhibits lower post-use strength when exposed to tap water whereas current flushable moist wipes take time and/or agitation.

All flushable moist wipes currently sold today that use salt sensitive adhesive are required to ensure a binder is applied throughout the z-direction of the wipe to provide the required in-use strength characteristics demanded by the consumer. Other flushable moist wipes that do not use a binder require strong fiber bonds throughout the z-direction to ensure in-use strength is acceptable.

Unfortunately, these approaches to addressing the dispersibility problems provide unacceptable strength or products that do not disperse quickly enough. Thus, there is a need to provide a wet wipe that provides proper in-use strength for consumers, but disperses more like toilet paper to pass various municipal regulations and be defined as a flushable product.

A dispersible wet wipe with the features of the preamble to claim 1 is disclosed in WO 2008/132614. Other dispersible wet wipes are disclosed in WO 2006/011932 and US 2004/058606.

### SUMMARY

The present invention provides a dispersible wet wipe in accordance with claim 1.

The present disclosure generally relates to single-ply dispersible wet wipes. More particularly, the disclosure relates to single-ply dispersible wet wipes constructed from a single-ply wipe substrate containing a fibrous substrate and a binder composition. The binder composition is applied substantially to the outer surfaces of the fibrous substrate. The wet wipes also contain a wetting composition containing between about 0.5 and about 3.5 percent of an insolubilizing agent, such as salt. Upon contact with agitated tap water for a period of ten minutes, the single-ply wipe substrate splits into two sections to enhance the dispersibility of the product.

In an exemplary embodiment, about 75 percent of the binder composition is distributed within one third of the outer surfaces of the fibrous substrate in a z-direction. In other words, less than 25 percent of the binder composition is distributed in the middle third layer of the fibrous substrate. More desirably, about 85 percent of the binder composition is distributed within one third of the outer surfaces 16, 18 of the fibrous substrate in a z-direction. In other words, less than 15 percent of the binder composition is distributed in the middle third layer of the fibrous substrate.

In exemplary embodiments, the fibrous substrates may have an air permeability of between 0.283 and 2.83 m³ per minute (10 and 100 cubic feet per minute).

The construction of the dispersible wipes may allow for a pass through percentage value of at least about 70 percent. More desirably, the single-ply dispersible wet wipes may have a pass through percentage value of at least about 95 percent. For purposes herein, the "pass through percentage value" is equal to the amount of the substrate that passes through the 3.18 mm perforated plate using the Dispersibility Shake Flask Test described herein.

The amount of binder composition present in the single-ply wipe substrates may desirably range from about 1 to about 15 percent by weight based on the total weight of the single-ply wipe substrates. More desirably, the binder composition may range from about 1 to about 8 percent by weight based on the total weight of the single-ply wipe substrate.

In exemplary embodiments, the wipes substrate is constructed from a fibrous substrate that may be a tissue web. In some embodiments, the tissue web may be an uncreped through-air dried tissue web.

Desirably, the wet wipes, as disclosed herein, may possess an in-use wet tensile strength of at least about 118 grams per linear cm (300 grams per linear inch). The sections of the dispersible wet wipe that have broken apart when soaked in tap water after about ten minutes or less have an after-use machine direction tensile strength of less than about 78.7 grams per linear cm (200 grams per linear inch).

### BRIEF DESCRIPTION

The features, aspects, and advantages of the present invention will become better understood with regard to the following description, appended claims, and accompanying drawings in which:
Figure 1 is a cross-sectional view of one embodiment of a single-ply wipe substrate described herein.
Figure 2 is a cross-sectional view of the dry substrate product illustrated in Figure 1 that has broken into two sections.

Repeated use of reference characters in the specification and drawings is intended to represent the same or analogous features or elements of the invention in different embodiments.

### DETAILED DESCRIPTION

The present disclosure generally relates to single-ply dispersible wet wipes. A single-ply dispersible wet wipe constructed from a single-ply wipe substrate containing a fibrous substrate and a binder composition is disclosed. The binder composition may be applied substantially to the outer surfaces of the fibrous substrate. The wet wipes also contain a wetting composition containing between about 0.5 and about 3.5 percent of an insolubilizing agent, such as salt. Upon contact with tap water agitated in a slosh box for a period of ten minutes, the single-ply wipe substrate splits into two sections to enhance the dispersibility of the product.

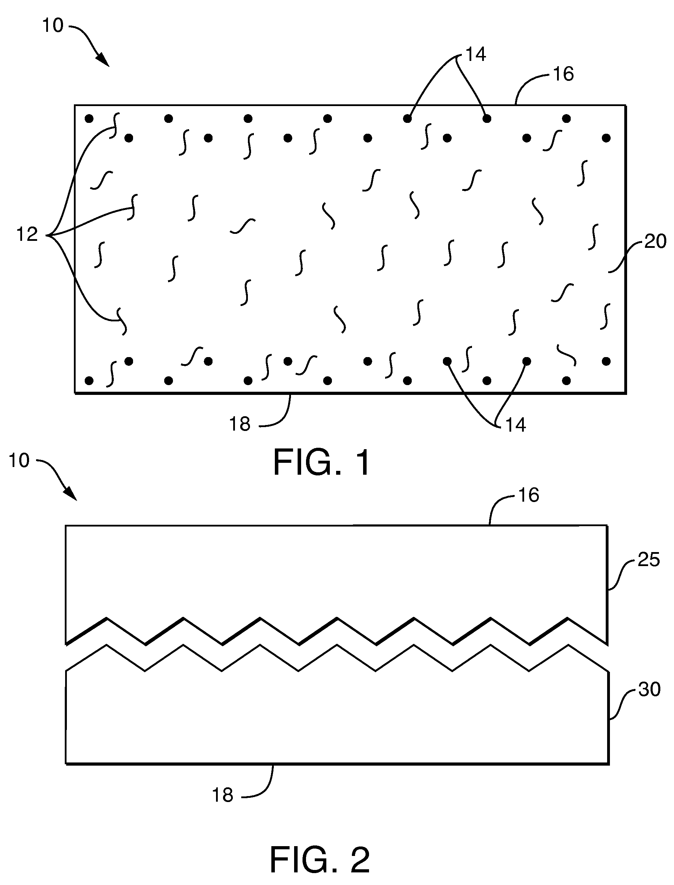

Referring to Figure 1, a dispersible wet wipe 10 is shown. The dispersible wipe is a single layer that includes a plurality of short dense fibers 12 and a binder composition 14. The binder composition 14 may be applied substantially to the outer surfaces 16, 18 of the fibrous substrate. For purposes of this disclosure, "applied substantially to the outer surfaces of the fibrous substrate" means that the majority of the binder composition is provided towards the outer surfaces 16, 18 of the single-ply wipe substrate. Providing a majority of the binder composition towards the outer surfaces 16, 18 of the single-ply wipe substrate enhances the dispersibility. This allows the single-ply wipe substrate to split into two sections to enhance the dispersibility of the product as illustrated in Figure 2. Desirably, about 75 percent of the binder composition is distributed within one third of the outer surfaces 16, 18 of the fibrous substrate in a z-direction. More desirably, about 85 percent of the binder composition is distributed within one third of the outer surfaces 16, 18 of the fibrous substrate in a z-direction.

When exposing this wipe to agitated tap water (such as a Slosh Box Test as described in the Test Procedures section herein), the wipe quickly breaks into two different sections that exhibit half the strength of the original substrate. This significantly reduces the time for product breakup and reduces issues with waste water treatment. Also, by breaking into two sections, more of the binder is exposed to the tap water which further improves binder degradation. Thus, the product loses strength rapidly after flushing and quickly breaks up in the wastewater system.

The composition of tap water can vary greatly depending on the water source. In the case of a dispersible wipe, the binder composition may preferably be capable of losing sufficient strength to allow the wet wipe to disperse in tap water covering the preponderance of the tap water composition range found throughout the United States (and throughout the world). Thus, it is important to evaluate the dispersibility of the binder composition in aqueous solutions which contain the major components in tap water and in a representative concentration range encompassing the majority of the tap water sources in the United States. The predominant inorganic ions typically found in drinking water are sodium, calcium, magnesium, bicarbonate, sulfate and chloride. Based on a recent study conducted by the American Water Works Association (AWWA) in 1996, the predominance of the U.S. municipal water systems (both ground water and surface water sources) surveyed have a total dissolved solids of inorganic components of about 500 ppm or less. This level of 500 ppm total dissolved solids also represents the secondary drinking water standard set by the U.S. Environmental Protection Agency. The average water hardness, which represents the calcium and magnesium concentrations in the tap water source, at this total dissolved solids level was approximately 250 ppm (CaCO₃ equivalent), which also encompasses the water hardness for the predominance of the municipal water systems surveyed by the AWWA. As defined by the United States Geological Survey (USGS), a water hardness of 250 ppm CaCO₃ equivalent would be considered "very hard" water. Similarly, the average bicarbonate concentration at 500 ppm total dissolved solids reported in the study was approximately 12 ppm, which also encompasses the bicarbonate, or alkalinity, of the predominance of the municipal water systems surveyed. A past study by the USGS of the finished water supplies of 100 of the largest cities in the United States suggests that a sulfate level of about 100 ppm is sufficient to cover the majority of finished water supplies. Similarly, sodium and chloride levels of at least 50 ppm each should be sufficient to cover the majority of U.S. finished water supplies. Thus, binder compositions which are capable of losing strength in tap water compositions meeting these minimum requirements should also lose strength in tap water compositions of lower total dissolved solids with varied compositions of calcium, magnesium, bicarbonate, sulfate, sodium, and chloride. To ensure the dispersibility of the binder composition across the country (and throughout the whole world), the binder composition may desirably be soluble in water containing up to about 100 ppm total dissolved solids and a CaCO₃ equivalent hardness up to about 55 ppm. More desirably, the binder composition may be soluble in water containing up to about 300 ppm of total dissolved solids and a CaCO₃ equivalent hardness up to about 150 ppm. Even more desirably, the binder composition may be soluble in water containing up to about 500 ppm total dissolved solids and a CaCO₃ equivalent hardness up to about 250 ppm.

In previously manufactured dispersible wet wipes, the binder composition is dispersed throughout the entire substrate. While this provides high strength, the plies of the prior dispersible wipes do not easily split into two sections, and thus do not disperse as well. A product disclosed herein having the binder composition "applied substantially to the outer surfaces of the fibrous substrate which splits into two sections loses strength rapidly after flushing and then breaks up in the wastewater system mitigating risk of clogging in sewers/drain line and providing complete passage of the flushability assessments as defined in INDA/EDANA guidelines.

The binder composition is provided substantially on the outer surfaces of the fibrous substrates by using little or no vacuum to draw the spray though the sheet, using a relatively less permeable sheet to minimize airflow through the sheet, and using small amounts of binder composition.

Desirably, the wet wipes, as disclosed herein, may possess an in-use wet tensile strength of at least about 0.12 N/mm (300 gf/in) when wetted with 10 to 400 percent of the wetting composition by weight relative to the weight of the wet wipe. The sections of the dispersible wet wipe that have broken apart when agitated in water with a total dissolved solids up to 500 ppm and a CaCO₃ equivalent hardness up to about 250 ppm after about 20 minutes or less have a post-use strength machine tensile strength of less than about 78.7 grams per linear cm (200 grams per linear inch).

Suitable materials for use in the single-ply wipe substrates may include fibrous sheet materials which include tissue webs, meltblown, airlaid, bonded-carded web materials, hydroentangled materials, and combinations thereof. Such materials can be comprised of synthetic or natural fibers, or a combination thereof.

Desirably, the single-ply wipe substrates are constructed from tissue webs. Basesheets suitable for this purpose can be made using any process that produces a low density, resilient tissue structure. Such processes include uncreped throughdried, creped throughdried and modified wet press processes. Exemplary patents include U.S. Patent No. 5,607,551, issued Mar. 4, 1997 to Farrington et al., U.S. Patent No. 5,672,248, issued Sep. 30, 1997 to Wendt et al., and U.S. Patent No. 5,593,545, issued Jan. 14, 1997 to Rugowski et al. U.S. Patent No. 6,083,346 issued Jul. 4, 2000 to Hermans et al, and U.S. Patent No. 7056572, issued Jan. 6, 2006 to Smith et al.. Typically, the tissue webs of the present disclosure define a basis weight of from about 60 to about 120 grams per square meter (gsm) and desirably from about 60 to about 90 gsm. Most desirably, the wipes of the present disclosure define a basis weight from about 65 to about 80 gsm.

For example, the tissue web may be made using an uncreped through-air-dried tissuemaking process in which a single-layer headbox deposits an aqueous suspension of papermaking fibers between forming wires. The newly-formed web is transferred from the forming wire to a slower moving transfer fabric with the aid of a vacuum box. The web is then transferred to a throughdrying fabric and passed over throughdryers to dry the web. After drying, the web is transferred from the throughdrying fabric to a reel fabric and thereafter briefly sandwiched between fabrics. The dried web remains with fabric until it is wound up into a parent roll.

Use of an uncreped through-air-dried tissue web may also provide benefits to the basesheet that allow for the binder to be distributed closer to the outer surfaces. As described above, using a relatively less permeable sheet to minimize airflow through the sheet will cause the binder to be present closer to the outer surfaces of the substrate. This allows for the sheet to break into two sections within the middle portion of the fibrous substrate. Desirably, the fibrous substrates may have an air permeability of between 0.283 and 2.83 m³ per minute (10 and 100 cubic feet per minute). More desirably, the fibrous substrates may have an air permeability of between 0.283 and 0.849 m³ per minute (10 and 30 cubic feet per minute).

Other materials suitable for the single-ply wipe substrates of the wipes are well know to those skilled in the art, and are typically made from a fibrous sheet material which may be either woven or nonwoven. Two types of wipe substrates are described herein, the "nonwoven fabrics" and the "nonwoven webs". The nonwoven material may comprise either a nonwoven fabric or a nonwoven web. The nonwoven fabric may comprise a fibrous material, while the nonwoven web may comprise the fibrous material and a binder composition. In another embodiment, as used herein, the nonwoven fabric comprises a fibrous material or substrate, where the fibrous material or substrate comprises a sheet that has a structure of individual fibers or filaments randomly arranged in a mat-like fashion, and does not include the binder composition. Since nonwoven fabrics do not include a binder composition, the fibrous substrate used for forming the nonwoven fabric may desirably have a greater degree of cohesiveness and/or tensile strength than the fibrous substrate that is used for forming the nonwoven web. For this reason nonwoven fabrics comprising fibrous substrates created via hydroentangling may be particularly preferred for formation of the nonwoven fabric. Hydroentangled fibrous materials may provide the desired in-use strength properties for wet wipes that comprise a nonwoven fabric.

In another embodiment, the single-ply wipe substrates may be an airlaid nonwoven fabric. The basis weights for airlaid nonwoven fabrics may range from about 20 to about 200 gsm with staple fibers having a denier of about 0.5 to 10 and a length of about 6 to 15 mm. Wet wipes may generally have a fiber density of about 0.025 to about 0.2 g/cm³. Wet wipes may generally have a basis weight of about 20 to about 150 gsm. More desirably the basis weight may be from about 30 to about 90 gsm. Even more desirably the basis weight may be from about 50 to about 75 gsm.

In an exemplary embodiment, the single-ply wipe substrates may be a nonwoven web. The nonwoven web may include the fibrous material and a binder composition. With regard to the nonwoven web, the binder composition may be applied to the fibrous material or substrate to form the nonwoven web using a variety of techniques. The fibrous material used to form the nonwoven web, may desirably have a relatively low wet cohesive strength prior to its treatment with the binder composition. Thus, in the case of a dispersible nonwoven web, when the fibrous substrate is bonded together by the binder composition, the nonwoven web will preferably break apart when it is placed in tap water, such as found in toilets and sinks. Thus the identity of the fibrous material may depend on whether it is to be used to form the nonwoven fabric or the nonwoven web. Furthermore, the fibers from which the fibrous material is made may also be selected based on whether they are to be used for a nonwoven web or nonwoven fabric. The fibers forming the fibrous material may be made from a variety of materials including natural fibers, synthetic fibers, and combinations thereof. The choice of fibers may depend upon, for example, the intended end use of the finished substrate, the fiber cost and whether fibers will be used for a nonwoven fabric or a nonwoven web. For instance, suitable fibers may include, but are not limited to, natural fibers such as cotton, linen, jute, hemp, wool, wood pulp, etc. Similarly, suitable fibers may also include: regenerated cellulosic fibers, such as viscose rayon and cuprammonium rayon; modified cellulosic fibers, such as cellulose acetate; or synthetic fibers, such as those derived from polypropylenes, polyethylenes, polyolefins, polyesters, polyamides, polyacrylics, etc. Regenerated cellulose fibers, as briefly discussed above, include rayon in all its varieties as well as other fibers derived from viscose or chemically modified cellulose, including regenerated cellulose and solvent-spun cellulose, such as Lyocell®. Among wood pulp fibers, any known papermaking fibers may be used, including softwood and hardwood fibers. Fibers, for example, may be chemically pulped or mechanically pulped, bleached or unbleached, virgin or recycled, high yield or low yield, and the like. Chemically treated natural cellulosic fibers may be used, such as mercerized pulps, chemically stiffened or crosslinked fibers, or sulfonated fibers.

In addition, cellulose produced by microbes and other cellulosic derivatives may be used. As used herein, the term "cellulosic" is meant to include any material having cellulose as a major constituent, and, specifically, comprising at least 50 percent by weight cellulose or a cellulose derivative. Thus, the term includes cotton, typical wood pulps, non-woody cellulosic fibers, cellulose acetate, cellulose triacetate, rayon, thermomechanical wood pulp, chemical wood pulp, debonded chemical wood pulp, milkweed, or bacterial cellulose. Blends of one or more of any of the previously described fibers may also be used, if so desired.

As described above, the single-ply wipe substrates include a binder composition. The binder composition includes a triggerable polymer. In another embodiment, the binder composition may comprise the triggerable polymer and a cobinder polymer.

As mentioned above, use of less binder composition allows more of the binder to remain near the surface of the fibrous substrate. The amount of binder composition add-on present in the single-ply wipe substrates may desirably range from about 1 to about 15 percent by weight based on the total weight of the single-ply wipe substrates. More desirably, the binder composition add-on may comprise from about 1 to about 10 percent by weight based on the total weight of the single-ply wipe substrates. Even more desirably, the binder composition add-on may comprise from about 1 to about 8 percent by weight based on the total weight of the single-ply wipe substrates. Most desirably, the binder composition add-on may comprise from about 3 to about 8 percent by weight based on the total weight of the single-ply wipe substrates. The amount of the binder composition desirably results in a single-ply wipe substrate that has in-use integrity, but quickly disperses when soaked in tap water.

As previously discussed, the binder composition may comprise the triggerable polymer and a cobinder. A variety of triggerable polymers may be used. One type of triggerable polymer is a dilution triggerable polymer. Examples of dilution triggerable polymers include ion-sensitive polymers, which may be employed in combination with a wetting composition in which the insolubilizing agent is a salt. Other dilution triggerable polymers may also be employed, wherein these dilution triggerable polymers are used in combination with wetting agents using a variety of insolubilizing agents, such as organic or polymeric compounds.

Although the triggerable polymer may be selected from a variety of polymers, including temperature sensitive polymers and pH-sensitive polymers, the triggerable polymer may preferably be the dilution triggerable polymer, comprising the ion-sensitive polymer. If the ion-sensitive polymer is derived from one or more monomers, where at least one contains an anionic functionality, the ion-sensitive polymer is referred to as an anionic ion-sensitive polymer. If the ion-sensitive polymer is derived from one or more monomers, where at least one contains a cationic functionality, the ion-sensitive polymer is referred to as a cationic ion-sensitive polymer. An exemplary anionic ion-sensitive polymer is described in U.S. Patent No. 6,423,804.

Examples of cationic ion-sensitive polymers are disclosed in the following U.S. Patent Application Publication Nos.: 2003/0026963, 2003/0027270, 2003/0032352, 2004/0030080, 2003/0055146, 2003/0022568, 2003/0045645, 2004/0058600, 2004/0058073, 2004/0063888, 2004/0055704, 2004/0058606, and 2004/0062791.

Desirably, the ion-sensitive polymer may be insoluble in the wetting composition, wherein the wetting composition comprises at least about 0.3 weight percent of an insolubilizing agent which may be comprised of one or more inorganic and/or organic salts containing monovalent and/or divalent ions. More desirably, the ion-sensitive polymer may be insoluble in the wetting composition, wherein the wetting composition comprises from about 0.3 to about 3.5 percent by weight of an insolubilizing agent which may be comprised of one or more inorganic and/or organic salts containing monovalent and/or divalent ions. Even more desirably, the ion-sensitive polymer may be insoluble in the wetting composition, wherein the wetting composition comprises from about 0.5 to about 3.5 percent by weight of an insolubilizing agent which comprises one or more inorganic and/or organic salts containing monovalent and/or divalent ions. Especially desirably, the ion-sensitive polymer may be insoluble in the wetting composition, wherein the wetting composition comprises from about 1 to about 3 percent by weight of an insolubilizing agent which comprises one or more inorganic and/or organic salts containing monovalent and/or divalent ions. Suitable monovalent ions include, but are not limited to, Na⁺ ions, K⁺ ions, Li⁺ ions, NH₄⁺ ions, low molecular weight quaternary ammonium compounds (e.g., those having fewer than five carbons on any side group), and a combination thereof. Suitable divalent ions include, but are not limited to, Zn²⁺, Ca²⁺ and Mg²⁺. These monovalent and divalent ions may be derived from organic and inorganic salts including, but not limited to, NaCl, NaBr, KCI, NH₄Cl, Na₂SO₄, ZnCl₂, CaCl₂, MgCl₂, MgSO₄, and combinations thereof. Typically, alkali metal halides are the most desirable monovalent or divalent ions because of cost, purity, low toxicity and availability. A desirable salt is NaCl.

In a preferred embodiment, the ion-sensitive polymer may desirably provide the nonwoven web with sufficient in-use strength (typically >0.115 N/mm (>300 gf/in)) in combination with the wetting composition containing sodium chloride. These nonwoven webs may be dispersible in tap water, desirably losing most of their wet strength (<0.0386 N/mm (<100 gf/in)) in 24 hours or less.

In another preferred embodiment, the ion-sensitive polymer may comprise the cationic sensitive polymer, wherein the cationic sensitive polymer is a cationic polyacrylate that is the polymerization product of 96 mol% methyl acrylate and 4 mol% [2-(acryloyloxy)ethyl]trimethyl ammonium chloride.

As previously discussed, the binder composition may comprise the triggerable polymer and/or the cobinder. When the binder composition comprises the triggerable polymer and the cobinder, the triggerable polymer and the cobinder may preferably be compatible with each other in aqueous solutions to: 1) allow for facile application of the binder composition to the fibrous substrate in a continuous process and 2) prevent interference with the dispersibility of the binder composition. Therefore, if the triggerable polymer is the anionic ion-sensitive polymer, cobinders which are anionic, nonionic, or very weakly cationic may be preferred. If the triggerable polymer is the cationic ion-sensitive polymer, cobinders which are cationic, nonionic, or very weakly anionic may be added. Additionally, the cobinder desirably does not provide substantial cohesion to the nonwoven material by way of covalent bonds, such that it interferes with the dispersibility of the nonwoven web.

The presence of the cobinder may provide a number of desirable qualities. For example, the cobinder may serve to reduce the shear viscosity of the triggerable polymer, such that the binder composition has improved sprayability over the triggerable binder alone. By use of the term "sprayable" it is meant that these polymers may be applied to the fibrous material or substrate by spraying, allowing the uniform distribution of these polymers across the surface of the substrate and penetration of these polymers into the substrate. The cobinder may also reduce the stiffness of the nonwoven web compared to the stiffness of a nonwoven web to which only the triggerable polymer has been applied. Reduced stiffness may be achieved if the cobinder has a glass transition temperature, Tg, which is lower than the Tg of the triggerable polymer. In addition, the cobinder may be less expensive than the triggerable polymer and by reducing the amount of triggerable polymer needed, may serve to reduce the cost of the binder composition. Thus, it may be desirable to use the highest amount of cobinder possible in the binder composition such that it does not jeopardize the dispersibility and in-use strength properties of the wet wipe. In a preferred embodiment, the cobinder replaces a portion of the triggerable polymer in the binder composition and permits a given strength level to be achieved, relative to a wet wipe having approximately the same tensile strength but containing only the triggerable polymer in the binder composition, to provide at least one of the following attributes: lower stiffness, better tactile properties (e.g. lubricity or smoothness) or reduced cost.

In one embodiment, the cobinder present in the binder composition, relative to the mass of the binder composition, may be about 10 percent or less, more desirably about 15 percent or less, more desirably 20 percent or less, more desirably 30 percent or less, or more desirably about 45 percent or less. Exemplary ranges of cobinder relative to the solid mass of the binder composition may include from about 1 to about 45 percent, from about 25 to about 35 percent, from about 1 to about 20 percent and from about 5 to about 25 percent.

The cobinder may be selected from a wide variety of polymers, as are known in the art. For example, the cobinder may be selected from the group consisting of poly(ethylene-vinyl acetate), poly(styrene-butadiene), poly(styrene-acrylic), a vinyl acrylic terpolymer, a polyester latex, an acrylic emulsion latex, poly(vinyl chloride), ethylene-vinyl chloride copolymer, a carboxylated vinyl acetate latex, and the like. A variety of additional exemplary cobinder polymers are discussed in U.S. Patent No. 6,653,406 and U.S. Patent Application Publication No. 2003/00326963. Particularly preferred cobinders include Airflex® EZ123 and Airflex® 110.

To prepare the single ply wipe substrates described herein, the binder composition may be applied to the fibrous material by any known process. Desirably applying the binder composition is done by electrostatic spraying. Desirably, the amount of binder composition may be metered and distributed uniformly onto the fibrous material or may be non-uniformly distributed onto the fibrous material.

Once the binder composition is applied to the fibrous material, drying, if necessary, may be achieved by any conventional means. Once dry, the single ply wipe substrate may exhibit improved tensile strength when compared to the tensile strength of the untreated wet-laid or dry-laid fibrous material, and yet should have the ability to rapidly "fall apart" or disintegrate when placed in tap water.

For ease of application to the fibrous substrate, the binder composition may be dissolved in water, or in a non-aqueous solvent, such as methanol, ethanol, acetone, or the like, with water being the preferred solvent. The amount of binder dissolved in the solvent may vary depending on the polymer used and the fabric application. Desirably, the binder solution contains less than about 18 percent by weight of binder composition solids. More desirably, the binder solution contains less than 16 percent by weight of binder composition solids.

A number of techniques may be employed to manufacture the wet wipes. In one embodiment, these techniques may include the following steps:
1. Providing the fibrous material (e.g., an unbonded airlaid, a tissue web, a carded web, fluff pulp, etc.).
2. Applying the binder composition to both sides of the fibrous material, typically in the form of a liquid, suspension, or foam to provide the single ply wipe substrate without a below vacuum system to draw the binder into the middle section of the wipe substrate.
3. The single ply wipe substrate may be dried.
4. Applying a wetting composition to the single ply wipe substrate to generate the wet wipe.
5. Placing the wet wipe in roll form or in a stack and packaging the product.

The binder composition as applied in step 2 comprises the triggerable polymer. In a further embodiment, the binder composition as applied in step 2 may comprise the triggerable polymer and the cobinder.

The finished wet wipes may be individually packaged, desirably in a folded condition, in a moisture proof envelope or packaged in containers holding any desired number of sheets in a water-tight package with a wetting composition applied to the wipe. Some example processes which can be used to manufacture folded wet wipes are described in U.S. Patent Nos. 5,540,332 and 6,905,748. The finished wipes may also be packaged as a roll of separable sheets in a moistureproof container holding any desired number of sheets on the roll with a wetting composition applied to the wipes. The roll can be coreless and either hollow or solid. Coreless rolls, including rolls with a hollow center or without a solid center, can be produced with known coreless roll winders, including those of SRP Industry, Inc. of San Jose, CA; Shimizu Manufacturing of Japan, and the devices disclosed in U.S. Patent No.

4,667,890. U.S. Patent No. 6,651,924 also provides examples of a process for producing coreless rolls of wet wipes.

In addition to the single ply wipe substrate, wet wipes also contain a wetting composition described herein. The wetting composition can be any liquid, which can be absorbed into the wet wipe basesheet and may include any suitable components, which provide the desired wiping properties. For example, the components may include water, emollients, surfactants, fragrances, preservatives, organic or inorganic acids, chelating agents, pH buffers, or combinations thereof, as are well known to those skilled in the art. Further, the liquid may also contain lotions, medicaments, and/or antimicrobials.

The wetting composition may desirably be incorporated into the wipe in an add-on amount of from about 10 to about 600 percent by weight of the substrate, more desirably from about 50 to about 500 percent by weight of the substrate, even more desirably from about 100 to about 500 percent by weight of the substrate, and especially more desirably from about 200 to about 300 percent by weight of the substrate.

In the case of a dispersible wipe, the wetting composition for use in combination with the nonwoven materials may desirably comprise an aqueous composition containing the insolubilizing agent that maintains the coherency of the binder composition and thus the in-use strength of the wet wipe until the insolubilizing agent is diluted with tap water. Thus the wetting composition may contribute to the triggerable property of the triggerable polymer and concomitantly the binder composition.

The insolubilizing agent in the wetting composition can be a salt, such as those previously disclosed for use with the ion-sensitive polymer, a blend of salts having both monovalent and multivalent ions, or any other compound, which provides in-use and storage strength to the binder composition and may be diluted in water to permit dispersion of the wet wipe as the binder composition transitions to a weaker state. The wetting composition may desirably contain more than about 0.3 weight percent of an insolubilizing agent based on the total weight of the wetting composition. The wetting composition may desirably contain from about 0.3 to about 3.5 weight percent of an insolubilizing agent based on the total weight of the wetting composition. More desirably, the wetting composition may contain from about 0.5 to about 3.5 weight percent of an insolubilizing agent based on the total weight of the wetting composition. More desirably, the wetting composition may contain from about 1 to about 3.5 weight percent of an insolubilizing agent based on the total weight of the wetting composition. Even more desirably, the wetting composition may contain from about 1 to about 2 weight percent of an insolubilizing agent based on the total weight of the wetting composition.

The wetting composition may desirably be compatible with the triggerable polymer, the cobinder polymer, and any other components of the binder composition. In addition, the wetting composition desirably contributes to the ability of the wet wipes to maintain coherency during use, storage and/or dispensing, while still providing dispersibility in tap water.

In one example, the wetting compositions may contain water. The wetting compositions can suitably contain water in an amount of from about 0.1 to about 99.9 percent by weight of the composition, more typically from about 40 to about 99 percent by weight of the composition, and more preferably from about 60 to about 99.9 percent by weight of the composition. For instance, where the composition is used in connection with a wet wipe, the composition can suitably contain water in an amount of from about 75 to about 99.9 percent by weight of the composition.

The wetting compositions may further contain additional agents that impart a beneficial effect on skin or hair and/or further act to improve the aesthetic feel of the compositions and wipes described herein. Examples of suitable skin benefit agents include emollients, sterols or sterol derivatives, natural and synthetic fats or oils, viscosity enhancers, rheology modifiers, polyols, surfactants, alcohols, esters, silicones, clays, starch, cellulose, particulates, moisturizers, film formers, slip modifiers, surface modifiers, skin protectants, humectants, sunscreens, and the like.

Thus, in one example, the wetting compositions may further optionally include one or more emollients, which typically act to soften, soothe, and otherwise lubricate and/or moisturize the skin. Suitable emollients that can be incorporated into the compositions include oils such as petrolatum based oils, petrolatum, mineral oils, alkyl dimethicones, alkyl methicones, alkyldimethicone copolyols, phenyl silicones, alkyl trimethylsilanes, dimethicone, dimethicone crosspolymers, cyclomethicone, lanolin and its derivatives, glycerol esters and derivatives, propylene glycol esters and derivatives, alkoxylated carboxylic acids, alkoxylated alcohols, and combinations thereof.

Ethers such as eucalyptol, cetearyl glucoside, dimethyl isosorbic polyglyceryl-3 cetyl ether, polyglyceryl-3 decyltetradecanol, propylene glycol myristyl ether, and combinations thereof, can also suitably be used as emollients.

In addition, the wetting composition may include an emollient in an amount of from about 0.01 to about 20 percent by weight of the composition, more desirably from about 0.05 to about 10 percent by weight of the composition, and more typically from about 0.1 to about 5 percent by weight of the composition.

One or more viscosity enhancers may also be added to the wetting composition to increase the viscosity, to help stabilize the composition thereby reducing migration of the composition and improving transfer to the skin. Suitable viscosity enhancers include polyolefin resins, lipophilic/oil thickeners, polyethylene, silica, silica silylate, silica methyl silylate, colloidal silicone dioxide, cetyl hydroxy ethyl cellulose, other organically modified celluloses, PVP/decane copolymer, PVM/MA decadiene crosspolymer, PVP/eicosene copolymer, PVP/hexadecane copolymer, clays, starches, gums, water-soluble acrylates, carbomers, acrylate based thickeners, surfactant thickeners, and combinations thereof.

The wetting composition may desirably include one or more viscosity enhancers in an amount of from about 0.01 to about 25 percent by weight of the composition, more desirably from about 0.05 to about 10 percent by weight of the composition, and even more desirably from about 0.1 to about 5 percent by weight of the composition.

The compositions of the disclosure may optionally further contain humectants. Examples of suitable humectants include glycerin, glycerin derivatives, sodium hyaluronate, betaine, amino acids, glycosaminoglycans, honey, sorbitol, glycols, polyols, sugars, hydrogenated starch hydrolysates, salts of PCA, lactic acid, lactates, and urea. A particularly preferred humectant is glycerin. The composition of the present disclosure may suitably include one or more humectants in an amount of from about 0.05 to about 25 percent by weight of the composition.

The compositions of the disclosure may optionally further contain film formers. Examples of suitable film formers include lanolin derivatives (e.g., acetylated lanolins), superfatted oils, cyclomethicone, cyclopentasiloxane, dimethicone, synthetic and biological polymers, proteins, quaternary ammonium materials, starches, gums, cellulosics, polysaccharides, albumen, acrylates derivatives, IPDI derivatives, and the like. The composition of the present disclosure may suitably include one or more film formers in an amount of from about 0.01 to about 20 percent by weight of the composition.

The wetting compositions may also further contain skin protectants. Examples of suitable skin protectants include ingredients referenced in SP monograph (21 CFR §347). Suitable skin protectants and amounts include those set forth in SP monograph, Subpart B - Active Ingredients §347.10: (a) Allantoin, 0.5 to 2%, (b) Aluminum hydroxide gel, 0.15 to 5%, (c) Calamine, 1 to 25%, (d) Cocoa butter, 50 to 100%, (e) Cod liver oil, 5 to 13.56%, in accordance with §347.20(a)(1) or (a)(2), provided the product is labeled so that the quantity used in a 24-hour period does not exceed 10,000 U.S.P. Units vitamin A and 400 U.S.P. Units cholecalciferol, (f) Colloidal oatmeal, 0.007% minimum; 0.003% minimum in combination with mineral oil in accordance with §347.20(a)(4), (g) Dimethicone, 1 to 30%, (h) Glycerin, 20 to 45%, (i) Hard fat, 50 to 100%, (j) Kaolin, 4 to 20%, (k) Lanolin, 12.5 to 50%, (I) Mineral oil, 50 to 100%; 30 to 35% in combination with colloidal oatmeal in accordance with §347.20(a)(4), (m) Petrolatum, 30 to 100%, (o) Sodium bicarbonate, (q) Topical starch, 10 to 98%, (r) White petrolatum, 30 to 100%, (s) Zinc acetate, 0.1 to 2%, (t) Zinc carbonate, 0.2 to 2%, (u) Zinc oxide, 1 to 25%.

The wetting compositions may also further contain sunscreens. Examples of suitable sunscreens include aminobenzoic acid, avobenzone, cinoxate, dioxybenzone, homosalate, menthyl anthranilate, octocrylene, octinoxate, octisalate, oxybenzone, padimate O, phenylbenzimidazole sulfonic acid, sulisobenzone, titanium dioxide, trolamine salicylate, zinc oxide, and combinations thereof. Other suitable sunscreens and amounts include those approved by the FDA, as described in the Final Over-the-Counter Drug Products Monograph on Sunscreens (Federal Register, 1999:64:27666-27693), herein incorporated by reference, as well as European Union approved sunscreens and amounts.

The wetting compositions may also further contain quaternary ammonium materials. Examples of suitable quaternary ammonium materials include polyquaternium-7, polyquaternium-10, benzalkonium chloride, behentrimonium methosulfate, cetrimonium chloride, cocamidopropyl pg-dimonium chloride, guar hydroxypropyltrimonium chloride, isostearamidopropyl morpholine lactate, polyquaternium-33, polyquaternium-60, polyquaternium-79, quaternium-18 hectorite, quaternium-79 hydrolyzed silk, quaternium-79 hydrolyzed soy protein, rapeseed amidopropyl ethyldimonium ethosulfate, silicone quaternium-7, stearalkonium chloride, palmitamidopropyltrimonium chloride, butylglucosides, hydroxypropyltrimonium chloride, laurdimoniumhydroxypropyl decylglucosides chloride, and the like. The composition of the present disclosure may suitably include one or more quaternary materials in an amount of from about 0.01 to about 20 percent by weight of the composition.

The wetting compositions may optionally further contain surfactants. Examples of suitable additional surfactants include, for example, anionic surfactants, cationic surfactants, amphoteric surfactants, zwitterionic surfactants, non-ionic surfactants, and combinations thereof. Specific examples of suitable surfactants are known in the art and include those suitable for incorporation into wetting compositions and wipes. The composition of the present disclosure may suitably include one or more surfactants in an amount of from about 0.01 to about 20 percent by weight of the composition.

In addition to nonionic surfactants, the cleanser may also contain other types of surfactants. For instance, in some embodiments, amphoteric surfactants, such as zwitterionic surfactants, may also be used. For instance, one class of amphoteric surfactants that may be used in the present disclosure are derivatives of secondary and tertiary amines having aliphatic radicals that are straight chain or branched, wherein one of the aliphatic substituents contains from about 8 to 18 carbon atoms and at least one of the aliphatic substituents contains an anionic water-solubilizing group, such as a carboxy, sulfonate, or sulfate group. Some examples of amphoteric surfactants include, but are not limited to, sodium 3-(dodecylamino)propionate, sodium 3-(dodecylamino)-propane-1-sulfonate, sodium 2-(dodecylamino)ethyl sulfate, sodium 2-(dimethylamino)octadecanoate, disodium 3-(N-carboxymethyl-dodecylamino)propane-1-sulfonate, disodium octadecyliminodiacetate, sodium 1-carboxymethyl-2-undecylimidazole, and sodium N,N-bis(2-hydroxyethyl)-2-sulfato-3-dodecoxypropylamine.

Additional classes of suitable amphoteric surfactants include phosphobetaines and the phosphitaines. For instance, some examples of such amphoteric surfactants include, but are not limited to, sodium coconut N-methyl taurate, sodium oleyl N-methyl taurate, sodium tall oil acid N-methyl taurate, sodium palmitoyl N-methyl taurate, cocodimethylcarboxymethylbetaine, lauryldimethylcarboxymethylbetaine, lauryldimethylcarboxyethylbetaine, cetyl-dimethylcarboxymethylbetaine, lauryl-bis-(2-hydroxyethyl)carboxymethyl-betaine, oleyldimethylgammacarboxypropylbetaine, lauryl-bis-(2-hydroxypropyl)-carboxy-ethylbetaine, cocoamidodimethylpropylsultaine, stearylamidodimethyl-propylsultaine, laurylamido-bis-(2-hydroxyethyl)propylsultaine, di-sodium oleamide PEG-2 sulfosuccinate, TEA oleamido PEG-2 sulfosuccinate, disodium oleamide MEA sulfosuccinate, disodium oleamide MIPA sulfosuccinate, disodium ricinoleamide MEA sulfosuccinate, disodium undecylenamide MEA sulfosuccinate, disodium lauryl sulfosuccinate, disodium wheat germamido MEA sulfosuccinate, disodium wheat germamido PEG-2 sulfosuccinate, disodium isostearamideo MEA sulfosuccinate, cocoamphoglycinate, cocoamphocarboxyglycinate, lauroampho-glycinate, lauroamphocarboxyglycinate, capryloamphocarboxyglycinate, cocoamphopropionate, cocoamphocarboxypropionate, lauroamphocarboxy-propionate, capryloamphocarboxypropionate, dihydroxyethyl tallow glycinate, cocoamido disodium 3-hydroxypropyl phosphobetaine, lauric myristic amido disodium 3-hydroxypropyl phosphobetaine, lauric myristic amido glyceryl phosphobetaine, lauric myristic amido carboxy disodium 3-hydroxypropyl phosphobetaine, cocoamido propyl monosodium phosphitaine, cocamidopropyl betaine, lauric myristic amido propyl monosodium phosphitaine, and mixtures thereof.

In certain instances, it may also be desired to utilize one or more anionic surfactants within the cleansers. Suitable anionic surfactants include, but are not limited to, alkyl sulfates, alkyl ether sulfates, alkyl ether sulfonates, sulfate esters of an alkylphenoxy polyoxyethylene ethanol, alpha-olefin sulfonates, beta-alkoxy alkane sulfonates, alkylauryl sulfonates, alkyl monoglyceride sulfates, alkyl monoglyceride sulfonates, alkyl carbonates, alkyl ether carboxylates, fatty acid salts, sulfosuccinates, sarcosinates, octoxynol or nonoxynol phosphates, taurates, fatty taurides, fatty acid amide polyoxyethylene sulfates, isethionates, or mixtures thereof.

Particular examples of some suitable anionic surfactants include, but are not limited to, C₈₋₁₈ alkyl sulfates, C₈₋₁₈ fatty acid salts, C₈₋₁₈ alkyl ether sulfates having one or two moles of ethoxylation, C₈₋₁₈ alkoyl sarcosinates, C₈₋₁₈ sulfoacetates, C₈₋₁₈ sulfosuccinates, C₈₋₁₈ alkyl diphenyl oxide disulfonates, C₈₋₁₈ alkyl carbonates, C₈₋₁₈ alpha-olefin sulfonates, methyl ester sulfonates, and blends thereof. The C₈₋₁₈ alkyl group can be straight chain (e.g., lauryl) or branched (e.g., 2-ethylhexyl). The cation of the anionic surfactant can be an alkali metal (e.g., sodium or potassium), ammonium, C₁₋₄ alkylammonium (e.g., mono-, di-, tri-), or C₁₋₃ alkanolammonium (e.g., mono-, di-, tri-).

Specific examples of such anionic surfactants include, but are not limited to, lauryl sulfates, octyl sulfates, 2-ethylhexyl sulfates, decyl sulfates, tridecyl sulfates, cocoates, lauroyl sarcosinates, lauryl sulfosuccinates, linear C₁₀ diphenyl oxide disulfonates, lauryl sulfosuccinates, lauryl ether sulfates (1 and 2 moles ethylene oxide), myristyl sulfates, oleates, stearates, tallates, ricinoleates, cetyl sulfates, and similar surfactants.

Cationic surfactants, such as cetylpyridinium chloride and methyl-benzethonium chloride, may also be utilized.

The wetting compositions may also further contain additional emulsifiers. As mentioned above, the natural fatty acids, esters and alcohols and their derivatives, and combinations thereof, may act as emulsifiers in the composition. Optionally, the composition may contain an additional emulsifier other than the natural fatty acids, esters and alcohols and their derivatives, and combinations thereof. Examples of suitable emulsifiers include nonionic emulsifiers such as polysorbate 20, polysorbate 80, anionic emulsifiers such as DEA phosphate, cationic emulsifiers such as behentrimonium methosulfate, and the like. The composition of the present disclosure may suitably include one or more additional emulsifiers in an amount of from about 0.01 to about 10 percent by weight of the composition.

For example, nonionic surfactants may be used as an emulsifier. Nonionic surfactants typically have a hydrophobic base, such as a long chain alkyl group or an alkylated aryl group, and a hydrophilic chain comprising a certain number (e.g., 1 to about 30) of ethoxy and/or propoxy moieties. Examples of some classes of nonionic surfactants that can be used include, but are not limited to, ethoxylated alkylphenols, ethoxylated and propoxylated fatty alcohols, polyethylene glycol ethers of methyl glucose, polyethylene glycol ethers of sorbitol, ethylene oxide-propylene oxide block copolymers, ethoxylated esters of fatty (C₈₋₁₈) acids, condensation products of ethylene oxide with long chain amines or amides, condensation products of ethylene oxide with alcohols, and mixtures thereof.

Various specific examples of suitable nonionic surfactants include, but are not limited to, methyl gluceth-10, PEG-20 methyl glucose distearate, PEG-20 methyl glucose sesquistearate, C₁₁₋₁₅ pareth-20, ceteth-8, ceteth-12, dodoxynol-12, laureth-15, PEG-20 castor oil, polysorbate 20, steareth-20, polyoxyethylene-10 cetyl ether, polyoxyethylene-10 stearyl ether, polyoxyethylene-20 cetyl ether, polyoxyethylene-10 oleyl ether, polyoxyethylene-20 oleyl ether, an ethoxylated nonylphenol, ethoxylated octylphenol, ethoxylated dodecylphenol, ethoxylated fatty (C₈₋₂₂) alcohol, including 3 to 20 ethylene oxide moieties, polyoxyethylene-20 isohexadecyl ether, polyoxyethylene-23 glycerol laurate, PEG 80 sorbitan laurate, polyoxy-ethylene-20 glyceryl stearate, PPG-10 methyl glucose ether, PPG-20 methyl glucose ether, polyoxyethylene-20 sorbitan monoesters, polyoxyethylene-80 castor oil, polyoxyethylene-15 tridecyl ether, polyoxy-ethylene-6 tridecyl ether, laureth-2, laureth-3, laureth-4, PEG-3 castor oil, PEG 600 dioleate, PEG 400 dioleate, and mixtures thereof.

The wetting compositions may also further contain preservatives. Suitable preservatives for use in the present compositions may include, for instance, Kathon CG, which is a mixture of methylchloroisothiazolinone and methylisothiazolinone available from Rohm & Haas of Philadelphia, PA; Neolone 950®, which is methylisothiazolinone available from Rohm & Haas of Philadelphia, PA; DMDM hydantoin (e.g., Glydant Plus available from Lonza, Inc. of Fair Lawn, NJ); iodopropynyl butylcarbamate; benzoic esters (parabens), such as methylparaben, propylparaben, butylparaben, ethylparaben, isopropylparaben, isobutylparaben, benzylparaben, sodium methylparaben, and sodium propylparaben; 2-bromo-2-nitropropane-1,3-diol; benzoic acid; imidazolidinyl urea; diazolidinyl urea; and the like. Still other preservatives may include ethylhexylglycerin, phenoxyethanol caprylyl glycol, a blend of 1,2-hexanediol, caprylyl glycol and tropolone, and a blend of phenoxyethanol and tropolone.

The wetting compositions may additionally include adjunct components conventionally found in pharmaceutical compositions in their art-established fashion and at their art-established levels. For example, the compositions may contain additional compatible pharmaceutically active materials for combination therapy, such as antimicrobials, antioxidants, anti-parasitic agents, antipruritics, antifungals, antiseptic actives, biological actives, astringents, keratolytic actives, local anesthetics, anti-stinging agents, anti-reddening agents, skin soothing agents, and combinations thereof. Other suitable additives that may be included in the compositions of the present disclosure include colorants, deodorants, fragrances, perfumes, emulsifiers, anti-foaming agents, lubricants, natural moisturizing agents, skin conditioning agents, skin protectants and other skin benefit agents (e.g., extracts such as aloe vera and anti-aging agents such as peptides), solvents, solubilizing agents, suspending agents, wetting agents, humectants, pH adjusters, buffering agents, dyes and/or pigments, and combinations thereof.

The wet wipes, as disclosed herein, do not require organic solvents to maintain in-use strength, and the wetting composition may be substantially free of organic solvents. Organic solvents may produce a greasy after-feel and cause irritation in higher amounts. However, small amounts of organic solvents may be included in the wetting composition for different purposes other than maintaining in-use wet strength. In one embodiment, small amounts of organic solvents (less than about 1 percent) may be utilized as fragrance or preservative solubilizers to improve process and shelf stability of the wetting composition. The wetting composition may desirably contain less than about 5 weight percent of organic solvents, such as propylene glycol and other glycols, polyhydroxy alcohols, and the like, based on the total weight of the wetting composition. More desirably, the wetting composition may contain less than about 3 weight percent of organic solvents. Even more desirably, the wetting composition may contain less than about 1 weight percent of organic solvents.

The wet wipes, as disclosed herein, desirably may be made to have sufficient tensile strength, sheet-to-sheet adhesion, calculated per layer stack thickness and flexibility.

The wet wipes may be prepared using a wipe substrate with a fibrous material and a binder composition forming a nonwoven airlaid web. These wet wipes made with single ply wipe substrate may also be made to be usable without breaking or tearing, to be consumer acceptable, and provide problem-free disposal once disposed in a household sanitation system.

The wet wipe formed with a nonwoven web desirably may have a machine direction tensile strength ranging from at least about 0.115 to about 0.386 N/mm (300 to about 1000 gf/in). More desirably, the wet wipe may have a machine direction tensile strength ranging from at least about 0.115 to about 0.304 N/mm (300 to about 800 gf/in). Even more desirably, the wet wipe may have a machine direction tensile strength ranging from at least about 0.115 to about 0.232 N/mm (300 to about 600 gf/in). Most desirably, the wet wipe may have a machine direction tensile strength ranging from at least about 0.135 to about 0.212 N/mm (350 to about 550 gf/in).

The total basis weight of the wipe substrate, consisting of a single layer of wipe substrate in the final wet wipe product, is between 60 and 90 gsm.

As mentioned previously, the wet wipes formed from the wipe substrate, may be sufficiently dispersible so that they lose enough strength to break apart in tap water under conditions typically experienced in household or municipal sanitation systems. Also mentioned previously, the tap water used for measuring dispersibility should encompass the concentration range of the majority of the components typically found in the tap water compositions that the wet wipe would encounter upon disposal. Previous methods for measuring dispersibility of the wipe substrates, whether dry or pre-moistened, have commonly relied on systems in which the material was exposed to shear while in water, such as measuring the time for a material to break up while being agitated by a mechanical mixer. Constant exposure to such relatively high, uncontrolled shear gradients offers an unrealistic and overly optimistic test for products designed to be flushed in a toilet, where the level of shear is extremely weak or brief. Shear rates may be negligible, for example once the material enters a septic tank. Thus, for a realistic appraisal of wet wipe dispersibility, the test methods should simulate the relatively low shear rates the products will experience once they have been flushed in the toilet.

A Slosh Box Test, for example, should illustrate the dispersibility of the wet wipe after it is fully wetted with water from the toilet and where it experiences negligible shear, such as in a septic tank. As described above, the wipe will split into two sections when submerged in tap water and run in a low shear method such as the Slosh Box Test described herein for a minimal period of time. Desirably, after the wet wipes split into two sections when submerged in tap water and agitated in a slosh box for about ten minutes or less. The two sections also exhibit an after-use tensile strength of less than about 0.077 N/mm (200 gf/in).

As mentioned previously, the wet wipes formed from the singly-ply wipe substrate, may be sufficiently dispersible so that they lose enough strength to break apart in tap water under conditions typically experienced in household or municipal sanitation systems. Also mentioned previously, the tap water used for measuring dispersibility should encompass the concentration range of the majority of the components typically found in the tap water compositions that the wet wipe would encounter upon disposal. The Dispersability Shake Flask Test is the first of two options to assess the dispersability or physical break-up of a test product during its transport through building drainlines, sewage pumps, and sewer pipes in the INDA/EDANA Guidance Document for Assessing the Flushabliity of Nowoven Consumer Products. It simulates the physical forces acting to disintegrate the product during passage through sewage pumps or through sewer pipes. The whole product is placed in a flask containing tap water or raw wastewater and is mechanically shaken under specified conditions. The contents of the flask are passed through a series of screens with sizes of 12.70, 6.35, 3.18 and 1.59 mm and the various size fractions retained on the screens are weighed so that the extent of disintegration can be determined. Under this test, if greater than 95 percent of the product mass passes through a 3.18 mm sieve (perforated plate) after agitation for one hour, then it is deemed that the product will adequately disperse during sewer conveyance. For purposes herein, the pass through percentage value is equal to the amount of the wipe that passes though the 3.18 mm perforated plate after one-hour of agitation. Thus, wipes will be the necessary size or smaller to allow the pieces to pass through the bar screens typically found in municipal sanitary sewer treatment facilities and not cause problems or blockages in households.

The Dispersability Shake Flask Test illustrates the dispersibility of the wet wipe after it is fully wetted with water from the toilet and where it experiences typical forces during its transport through sewage pumps and municipal wastewater conveyance systems.

In one embodiment, the dispersible wet wipe has a pass through percentage value of at least 70 percent. More desirably, the dispersible wet wipe has a pass through percentage value of at least 90 percent. Even more desirably, the dispersible wet wipe has a pass through percentage value of at least 95 percent.

Most desirably, the wet wipes, as disclosed herein, may possess an in-use wet tensile strength greater than about 0.115 N/mm (300 gf/in). Additionally, after the wet wipes split into two section when submerged in tap water and agitated in a slosh box for about ten minutes or less, the two sections exhibit an after-use tensile strength of less than about 0.077 N/mm (200 gf/in).

The wet wipe preferably maintains its desired characteristics over the time periods involved in warehousing, transportation, retail display and storage by the consumer. In one embodiment, shelf life may range from two months to two years.

The wet wipes, as disclosed herein, are illustrated by the following examples, which are not to be construed in any way as imposing limitations upon the scope thereof. On the contrary, it is to be clearly understood that resort may be had to various other embodiments, modifications, and equivalents thereof, which, after reading the description herein, may suggest themselves to those skilled in the art without departing from the spirit and/or the scope of the appended claims.

### TEST METHODS

### Wet Wipe Tensile Strength Measurements

For purposes herein, tensile strength may be measured using a Constant Rate of Elongation (CRE) tensile tester using a 2.54 cm (1-inch) jaw width (sample width), a test span of 7.62 cm (3 inches) (gauge length), and a rate of jaw separation of 25.4 centimeters per minute after maintaining the sample at the ambient conditions of 23 ± 2°C and 50 ± 5% relative humidity for four hours before testing the sample at the same ambient conditions. The wet wipes are cut with 2.54 cm (1-inch) wide strips cut from the center of the wipes in the specified machine direction (MD) or cross-machine direction (CD) orientation using a JDC Precision Sample Cutter (Thwing-Albert Instrument Company, Philadelphia, PA, Model No. JDC 3-10, Serial No. 37333). The "MD tensile strength" is the peak load in grams-force per inch of sample width when a sample is pulled to rupture in the machine direction. The "CD tensile strength" is the peak load in grams-force per inch of sample width when a sample is pulled to rupture in the cross direction.

The instrument used for measuring tensile strength is an MTS Systems Sinergy 200 model. The data acquisition software is MTS TestWorks® for Windows Ver. 4.0 commercially available from MTS Systems Corp., Eden Prairie, MN. The load cell is an MTS 50 Newton maximum load cell. The gauge length between jaws is 7.62 ± 0.1 cm (3 ± 0.04 inches). The top and bottom jaws are operated using pneumatic-action with maximum 413 KPa (60 P.S.I.) The break sensitivity is set at 40 percent. The data acquisition rate is set at 100 Hz (i.e. 100 samples per second). The sample is placed in the jaws of the instrument, centered both vertically and horizontally. The test is then started and ends when the force drops by 40 percent of peak. The peak load expressed in grams-force is recorded as the tensile strength of the specimen. At least twelve representative specimens are tested for each product and its average peak load is determined. As used herein, the "geometric mean tensile strength" (GMT) is the square root of the product of the dry machine direction tensile strength multiplied by the 15 dry cross-machine direction tensile strength and is expressed as grams per inch of sample width. All of these values are for in-use tensile strength measurements.

To provide after-use tensile strength measurements, the samples are submerged in tap water and agitated in the Slosh Box Test allowed to split into two sections through the central portion of the wipe substrate. The two sections are then measured for MD and CD tensile strength.

### Basis Weight

The dry basis weight of the basesheet material forming the wet wipes in the stack can be obtained using the ASTM active standard D646-96(2001), Standard Test Method for Grammage of Paper and Paperboard (Mass per Unit Area), or an equivalent method.

### Dispersibility Shake Flask Test

The Percentage Mass Loss of the wet wipes can be obtained using the INDA/EDANA Guidance Document for Assessing the Flushability of Nonwoven Consumer Products, Dispersibilty Shake Flask Test. For purposes herein, samples were placed into tap water and tested after shaking on the flask shaker for one hour.

As used herein, the Pass Through Percentage Value is equal to Percentage Mass Loss, or the amount of the substrate that passes through the 3 mm perforated plate.

This test is used to assess the dispersibility or physical breakup of a flushable product during its transport through sewage pumps (e.g., ejector or grinder pumps) and municipal wastewater conveyance systems (e.g., sewer pipes and lift stations). This test assesses the rate and extent of disintegration of a test material in the presence of tap water or raw wastewater. Results from this test are used to predict the compatibility of a flushable product with household sewage pumps and municipal collection systems.

Materials and Apparatus:
1. Fernbach triple-baffled, glass, culture flasks (2800 mL).
2. Orbital floor shaker with 2-in (5-cm) orbit capable of 150 rpm. The platform for the shaker needs clamps to be able to accommodate a bottom flask diameter of 205 mm.
3. USA Standard Testing Sieve #18 (1 mm opening): 8 in (20 cm) diameter.
4. Perforated Plate Screens details

| **Hole Size (mm)** | **Hole size (in)** | **Hole Center** | **Pattern** | **Gauge** | **% open area** |
|---|---|---|---|---|---|
| 12.75 mm | 1/2" | 11/16" | Staggered | 16SWG | 48% |
| 6.35 mm | 1/4" | 5/16" | Staggered | 16SWG | 58% |
| 3.18 mm | 1/8" | 3/16" | Staggered | 20SWG | 40% |
| 1.59 mm | 1/16" | 3/32" | Staggered | 20SWG | 41% |

5. Drying oven capable of maintaining a temperature of 40 ± 3°C for thermoplastic test materials and capable of maintaining a temperature of 103 ± 3°C for non-plastic test materials.

### Test Initiation:

Each test product is run in triplicate so there are three flasks prepared for each of the two predetermined destructive sampling time points. Each flask contains one liter of prescreened wastewater or room temperature tap water and the test product (see section 6.1 Summary of Test Methods for guidance in choosing a medium for test). Each test product should be pre-weighed in triplicate (dry weight basis) on an analytical balance that measures at least 2-decimal places and then these weights recorded in a laboratory notebook for later use in the final percent disintegration calculations. Control flasks with the reference material are also run to accommodate two destructive sampling time points. Each flask also contains one liter of prescreened wastewater or tap water and an appropriate reference material. Whatman #41 ashless filter paper if used should be folded into quarters and reopened before placing in flask. For products that are pre-moistened (e.g., wet wipes) sample preconditioning to simulate product delivery to the sewer can be performed by flushing the product through the toilet and drain line apparatus. This should be documented in the study record. Measure one liter of wastewater or tap water into each of the Fernbach flasks and place them on the rotary shaker table. Add test product to the flasks (either one article or for toilet tissue typically 1 to 3 grams on dry weight basis). A minimum of one gram of test product should be used to ensure accurate measurement of the disintegration loss. The flasks are shaken at 150 rpm. For the sewage pump assessment test and control products are observed after 30 and 60 minutes, and then destructively sampled at three hours. For the sewer conveyance assessment, visual observations of the test and control products are made at one hour, and then destructively sampled at six hours. These tests are incubated at room temperature (22 ± 3°C).

### Test Termination:

At the designated destructive sampling points a flask from each set of products being tested and the control set is removed and the contents poured through a nest of screens arranged from top to bottom in the following order: 12 mm, 3 mm and 1.5 mm (diameter opening). Additional screens can be added to better understand the dispersibility characteristics of the sample. With a hand held showerhead spray nozzle held approximately 10 to 15 cm above the sieve, the material is gently rinsed through the nested screens for two minutes at a flow rate of 4 L/min being careful not to force passage of the retained material through the next smaller screen. After the two minutes of rinsing the top screen is removed and rinsing of the next smaller screen, still nested, continues for two additional minutes using the same procedure as above. The rinsing process is continued until all of the screens have been rinsed. After rinsing is complete, the retained material is removed from each of the screens using forceps or by backwashing into a smaller sized sieve. The content from each screen is transferred to a separate, labeled tared aluminum weigh pan and dried overnight at 103 ± 3°C. Dried samples are cooled in a desiccator. After cooling the materials are weighed and percentage of disintegration based on the initial starting weight of the test material is calculated.

### Slosh Box Test

This method uses a bench-scaled apparatus to evaluate the breakup or dispersibility of flushable consumer products as they travel through the wastewater collection system. In this test method, a clear plastic tank is loaded with a product and tap water or raw wastewater. The container is then moved up and down by a cam system at a specified rotational speed to simulate the movement of wastewater in the collection system. The initial breakup point and the time for dispersion of the product into pieces measuring 1 in x 1 in (25 mm x 25 mm) are recorded in the laboratory notebook. This 1 in x 1 in (25 mm x 25 mm) size is a parameter that is used because it reduces the potential of product recognition. The testing can be extended until the product is fully dispersed. The various components of the product are then screened and weighed to determine the rate and level of disintegration.

### Testing Parameters:

The slosh box water transport simulator consists of a transparent plastic tank that is mounted on an oscillating platform with speed and holding time controller. The angle of incline produced by the cam system produces a water motion equivalent to 60 cm/s (2 ft/s), which is the minimum design standard for wastewater flow rate in an enclosed collection system. The rate of oscillation is controlled mechanically by the rotation of a cam and level system and should be measured periodically throughout the test. This cycle mimics the normal back-and forth movement of wastewater as it flows through sewer pipe.

### Test Initiation:

Room temperature tap water (softened and/or non-softened) or raw wastewater (2000 mL) is placed in the plastic container/tank. The timer is set for 6 hours (or longer) and cycle speed is set for 26 rpm. The pre-weighed product is placed in the tank and observed as it undergoes the agitation period. For toilet tissue, add a number of sheets that range in weight from 1 to 3 grams. All other products may be added whole with no more than one article per test. A minimum of one gram of test product is recommended so that adequate loss measurements can be made. The time to break up into two sections is recorded in the laboratory notebook.

### Dutch Pump Test

This test assesses the compatibility of products intended to be flushed with municipal sewage pumping systems. In this test, product is periodically introduced into the inlet of a continuously running sewage pump which is situated in a recirculating water tank. Each single test is run for a total of 60 test pieces which are introduced into the pump at a rate of one sample every ten seconds. The pump power consumption and flow rate on the outlet are continuously monitored and recorded. A flow rate corresponding to 100 percent efficiency point for the specified pump is used.

### Test System Preparation:

The tank needs to be filled with water up to the 'fill mark' which is 60 mm below the top. Ensure that both the pump and valve outlet on the pipe are free from foreign material. Calibration of the flow meter needs to be conducted prior to testing and then periodically in accordance with the manufacturer's specification. Ensure that there is no remaining air in the circuit before starting the pump. Switch on the power to the pump and check for proper flow direction before setting flow rate using valve on the outlet pipe. Before beginning to test any product, switch on the pump; adjust the flow rate to 21.2 +/- 0.3 liters/sec and run the pump for 30 minutes. This allows the pump motor temperature to become stabilized and hence consume power at a steady rate. If testing is to be carried out at widely spaced intervals, this needs to be repeated prior to each set of tests to ensure baseline power consumption is steady.

### Sample preparation:

Typical residence times for products to reach a municipal sewage pump are 1 to 3 hours from flushing. To best simulate the condition of products entering the sewage pump in the field, a 1 hour presoak for all products should be used. To presoak the test pieces fill both plastic buckets with room temperature tap water, place 30 test pieces of the product into each bucket, submerge the products into the water by hand with a gentle swirling action for 5 to 10 seconds. Leave products to stand for 60 minutes before starting the test sequence.

### Test Sequence:

Check that the flow rate is correct (21.2 +/- 0.3 liters/sec) and that the power consumption is stable within the range 1.95 +/- 0.05kW before introducing the first piece. If it is not, stop the pump, and perform tank cleaning as defined below to remove any debris from the circuit. Then start the pump again. Note the power consumption. Report the water temperature in the tank in °C. Start the data logger and the stop watch running. Using a loading device (i.e. broom stick or garbage picker) wait 10 seconds to load first test piece into the pump and then load all subsequent test pieces at 10 second intervals (measured using the stop watch) until all 60 test pieces have been introduced. During the introduction of the test pieces the flow rate has to be controlled and maintained at (21.2 ± 0.3) l/s (or 76.3 ± 1.1 m3/h). After the introduction of the last test piece let the pump run 10 additional seconds then stop the data logger. A total of 5 test runs of 60 test pieces each are required to be conducted for each product to complete the full test. Report the water temperature in °C at the end of a set of five replicates.

### Collection of Data/Measurements:

The following measurements and observations should be made during the test run:
1. Set Data capture at 1 second interval
2. Record power consumption data log during testing sequence (i.e. Kilowatts)
3. Calculate average power without product (Pstart). This is calculated as the average power value for 10 seconds of pump operation before any wipes are put into the pump.

### Data Manipulation:

Data capture files should be exported into Microsoft Excel/Labview or similar software to calculate percent power increase and generate graphical output. Power recordings have to be smoothed before calculation/graphical display. Smoothing is achieved by taking the average of data capture results recorded over each 5 seconds. These averages become P (1 to n) i.e. one 60 wipe test will have 120 points on the graph. Percent Power Increase is calculated for each power reading as ((**Pn/Pstart)** -1) x 100% For each single test run record the total number of data points, calculated P (1 to n) Identify Pn values where the Power increase is more than 10 percent. Calculate percent of total Pn values where power increase is >10 percent.

For a product to pass this test, Less than 10 percent of all Pn values over the five runs of 60 wipes can have power increase of more than 10 percent.

### Air Permeability

The Air Permeability is measured in cubic feet of air per minute passing through a 38 square cm area (circle with 7 cm diameter) using a Textest FX3300 air permeability tester manufactured by Textest Ltd., Zurich, Switzerland. All tests are conducted in a laboratory with a temperature of 23 +/- 2°C and 50 +/-5% RH. Specifically, the moist wipe sheet is allowed to dry out and condition for at least 12 hours in the 23 +/- 2°C and 50 +/- 5% RH laboratory before testing. The wipe is clamped in the 7 cm diameter sheet test opening and the tester is set to a pressure drop of 125 Pa. Placing folds or crimps above the fabric test opening is to be avoided if at all possible. The unit is turned on by applying clamping pressure to the sample. The air flow under the 125 Pa pressure drop is recorded after 15 seconds of airflow to achieve a steady state value.

### Binder Distribution in Z-Direction

The binder distribution method measures the amount of binder throughout the thickness or z-direction of a fibrous material. The measurement is performed using binder staining, cross-sectioning, and image analysis to detect and then measure the binder distribution in the z-direction when viewed using a microscope implementing transmitted light. An image analysis algorithm was developed to detect and measure the binder distribution in the z-direction, and the resulting histogram data were then used to measure the percentages of the binder in the top, middle and bottom one-third layers of a material's thickness. For example, four images from each of four adjacently cut cross-sections of a fibrous material (e.g. dried wet wipe) containing a binder were acquired and analyzed at several points along their z-axis to arrive at both total material and binder histograms which mathematically described the material and binder distributions in the z-direction. The histograms were then used to calculate the percentages of binder in each one-third of the thickness of the material. Typically, several such material subsamples are analyzed for a sample to arrive at mean binder percentage values.

### Detailed Method for Binder Distribution in the Z-direction Analysis

A tissue or similar fibrous sample is allowed to dry and equilibrate at laboratory temperature conditions ranging from 20 to 22°C (68 to 72°F), and a relative humidity between 45 to 55 percent for at least 24 hours.

### Sample Preparation:

Product packages were opened with scissors and individual sheets removed from the package maintaining the top-to-bottom orientation of the sheets. Random samples were selected for continued processing. Selected samples were allowed to air dry. After complete drying, 5.08 by 10.16 cm (two- by four-inch) subsamples were randomly cut from the sheets for staining.

### Staining:

The two by four-inch subsamples were stained with a 1%-weight/volume solution of FD&C Blue 1 (CAS #3844-45-9) in 0.9%-weight/volume saline. Subsamples were individually immersed in a volume of the staining solution that was at least ten times greater the volume of the subsample for five minutes. The staining solution was poured off and subsamples rinsed of unbound stain by five exchanges of tap water using the following schedule 30 seconds, 1 minute, 2 minutes, 2 minutes and 2 minutes. Stained subsamples were placed on blotter paper for five seconds, flipped to the other side for five seconds, and then hung vertically to air dry overnight.

### Infiltration and Embedding:

Stained and dried subsamples were cut with a single edge razor to fit inside 22 mm by 22 mm polypropylene embedding cups such as Peel-A-Way disposable plastic tissue embedding molds manufactured by Peel-A-Way Scientific, South EI Monte, CA. These 22-mm squares were mounted with the product top-surface up to card stock-frames with two staples. These assemblies were infiltrated and embedded in LR White as was obtained as a kit from Electron Microscopy Sciences, Hatfield, PA. The inner surfaces of the embedding cups were swabbed with LR White cold cure accelerator, the subsample-card stock assembles placed inside the cups, and the cups placed in an ice bath. The assemblies within the cups were infiltrated with LR White mixed with the cold-cure accelerator following the manufacturer's directions. Complete infiltration was facilitated by introduction of the embedding media at one corner of the assembly and allowing the natural wicking of material to draw the embedding media into itself. Infiltration was completed within five minutes and sufficient embedding media was added to cover the entire subsample assembly with at least 5 mm of fluid. Material remained in the ice bath while the resin hardened.

### Sectioning and Mounting:

Sections of the embedded subsamples were cut with a Reichert-Jung Polycut E sledge microtome at 20 micrometers. The sectioning protocol was to cut and discard thirty 10-micrometer sections and then collect four 20-micrometer sections in sequence. The four 20-micrometer sections were trimmed and mounted in sequence to a slide with low viscosity (150-cs) immersion oil (n=1.515) and cover slip. This procedure was repeated for a total of four slides and then this sectioning protocol was repeated with a new block and sectioning orientation to produce three additional slides.

### Analysis of Sections:

A Leica DFC 300 color video camera (Leica Microsystems Heerbrugg, Switzerland) is mounted on a Leitz DMRX microscope (Leica Mikroskopie, Wetzlar, Germany) fitted with a 5X objective lens and possessing an x-y-z motion auto-stage controllable with a manual joystick. The auto-stage is a motorized apparatus known to those skilled in the analytical arts which was purchased from Marzhauser Wetzlar, having an office in Wetzlar, Germany. The auto stage is used to move the microscope slide containing the four cross-sectional subsamples in order to obtain four separate and distinct, non-overlapping images, one from each section of the specimen. The stage also provided z-plane focusing capabilities. The slide is placed onto the auto-stage of the Leitz DMRX microscope in such a way that the top surface of the fibrous material as removed from its product container is also the top surface in the image. The sample is illuminated from beneath the auto-stage using the Leitz microscope transmitted light illumination system with the gray filter and low magnification light condenser in place.

The image analysis software platform used to acquire images and perform the binder distribution measurements may be a QWIN Pro (Version 3.2.1) available from Leica Microsystems, having an office in Heerbrugg, Switzerland. The custom-written image analysis algorithm 'Z-Binder Distribution - 1' is used to acquire, process and perform measurements of color images using Quantimet User Interactive Programming System (QUIPS) language. The custom image analysis algorithm is shown below.
NAME: Z-Binder Distribution - 1
PURPOSE: Measures z-distribution of stained binder on fibrous substrates
CONDITIONS: Leitz DMRX w/ 5X obj.; Transmitted light w/ gray filter; 20 um thick epoxy sections; Leica DFC 300 color vid.
DATE: May 5, 2010
AUTHOR: D.G. Biggs
SET-UP
Clear Accepts
Open File (C:\Data\22776\totdistribution.xls, channel #2)
Open File (C:\Data\22776\binderdistribution1.xls, channel #1)
   -- Calvalue = 1.29 um/pixel
CALVALUE = 1.29
ACQOUTPUT = 0
Calibrate (CALVALUE CALUNITS$ per pixel)
Measure frame (x 31, y 1, Width 1330, Height 1039)
Image frame (x 0, y 0, Width 1392, Height 1040)
Enter Results Header
File Results Header (channel #1)
File Line (channel #1)
File Results Header (channel #2)
File Line (channel #2)
For (IMAGE = 1 to 4, step 1)
Clear Feature Histogram #1
Clear Feature Histogram #3
DEFINE BINARY GRAPHICS VARIABLES
GRAPHORGX = 32
IMAGE ACQUISITION AND DETECTION
Display (Colour0 (on), frames (on,on), planes (off,off,off,off,off,off), lut 0, x 0, y 0, z 1, Reduction off)
Image Setup DC Twain [PAUSE] (Camera 1, AutoExposure Off, Gain 0.00, ExposureTime 34.23 msec, Brightness 0, Lamp 0.00)
Acquire (into Colour0)
Colour Transform (RGB to HSI, from Colour0 to Colour0) ACQFILE$ = "C:\Images\22776\UCTAD-7_"+STR$(IMAGE)+".JPG" Write image (from ACQOUTPUT into file ACQFILE$)
   -- Detect all material
Colour Detect (HSI+: 0-255, 0-255, 0-192, from Colour0 into Binary0)
IMAGE PROCESSING
PauseText ("Accept the primary structure and exclude any outlying debris.")
Binary Edit [PAUSE] (Accept from Binary0 to Binary1, nib Fill, width 2)
Binary Amend (Open from Binary1 to Binary1, cycles 1, operator Disc, edge erode on)
Binary Amend (Close from Binary1 to Binary2, cycles 120, operator Disc, edge erode on)
Binary Identify (FillHoles from Binary2 to Binary3)
Binary Amend (Open from Binary3 to Binary4, cycles 5, operator Disc, edge erode on)
BOLEAN AND MEASUREMENT
For (BINGRAPH = 1 to 26, step 1)
GRAPHORGY = 2
GRAPHNX = 1
GRAPHNY = 1
GRAPHWID = 50
GRAPHHGHT = 1038
GRAPHTHIK = 1
GRAPHORNT = 0
GRAPHOUT = 13
Graphics (Inverted Grid, GRAPHNX x GRAPHNY Lines, Grid Size GRAPHWID x GRAPHHGHT, Origin GRAPHORGX x GRAPHORGY, Thickness GRAPHTHIK, Orientation GRAPHORNT, to GRAPHOUT Cleared)
Binary Logical (C = A AND B: C Binary5, A Binary4, B Binary13)
CENTER YPOS
Measure feature (plane Binary5, 32 ferets, minimum area: 10, grey image: Colour0)
   Selected parameters: UserDef1, YCentroid
Feature Expression (UserDef1 (all features), title CalcA = (PYCENTROID(FTR)-520))
GREYUTILIN = 0
GREYUTILOUT = 1
   -- Shift Grey Image
If (PUSERDEF1 (FTR) < 0)
   DISTANCE = (PUSERDEF1 (FTR)**2)**0.5
   SHIFT.SIZE = DISTANCE
   SHIFT.DIRN = 270
   Grey Util (Shift GREYUTILIN to GREYUTILOUT by SHIFT.SIZE at SHIFT.DIRN degs)
Endif
If (PUSERDEF1(FTR)>0)
   DISTANCE = PUSERDEF1(FTR)
   SHIFT.SIZE = DISTANCE
   SHIFT.DIRN = 90
   Grey Util (Shift GREYUTILIN to GREYUTILOUT by SHIFT.SIZE at SHIFT.DIRN degs)
Endif
If (PUSERDEF1(FTR)=0)
   Grey Util (Copy Colour0 to Colour1)
Endif
Display (Colour1 (on), frames (on,on), planes (off,off,off,off,off,off), lut 0, x 0, y 0, z 1, Reduction off)
DETECT AFTER CENTERING
   - Detect binder
Colour Detect (HSI+: 0-74, 60-255, 0-255, from Colour1 into Binary10)
Binary Amend (Close from Binary10 to Binary10, cycles 1, operator Disc, edge erode on)
Binary Amend (Open from Binary10 to Binary11, cycles 1, operator Disc, edge erode on)
   - Detect all material
Colour Detect (HSI+: 0-255, 0-255, 0-192, from Colour1 into Binary0)
Binary Amend (Close from Binary0 to Binary0, cycles 1, operator Disc, edge erode on)
Binary Amend (Open from Binary0 to Binary0, cycles 1, operator Disc, edge erode on)
MEASURE BINDER Z-DISTRIBUTION
GRAPHORGY = 2
GRAPHNX = 1
GRAPHNY = 1
GRAPHWID = 50
GRAPHHGHT = 1038
GRAPHTHIK = 1
GRAPHORNT = 0
GRAPHOUT = 12
Graphics (Inverted Grid, GRAPHNX x GRAPHNY Lines, Grid Size GRAPHWID x GRAPHHGHT, Origin GRAPHORGX x GRAPHORGY, Thickness GRAPHTHIK, Orientation GRAPHORNT, to GRAPHOUT Cleared)
Binary Logical (C = A AND B: C Binary6, A Binary12, B Binary11) Measure feature (plane Binary6, 32 ferets, minimum area: 10, grey image: Image1)
   Selected parameters: Area, UserDef2, YCentroid
Feature Expression (UserDef2 (all features), title YFEAT =
PYCENTROID(FTR) *CALVALUE)
Feature Histogram #1 (Y Param Area, X Param UserDef2, from 0.
to 1340., linear, 40 bins)
Feature Histogram #2 (Y Param Area, X Param UserDef2, from 0.
to 1340., linear, 40 bins)
MEASURE TOTAL MATERIAL Z-DISTRIBUTION
Binary Logical (C = A AND B: C Binary7, A Binary12, B Binary0) Measure feature (plane Binary7, 32 ferets, minimum area: 10, grey image: Colour0)
   Selected parameters: Area, X FCP, Y FCP, UserDef2, YCentroid Feature Expression (UserDef2 (all features), title YFEAT =
PYCENTROID(FTR)* CALVALUE)
Feature Histogram #3 (Y Param Area, X Param UserDef2, from 0. to 1340., linear, 40 bins)
Feature Histogram #4 (Y Param Area, X Param UserDef2, from 0.
to 1340., linear, 40 bins)
GRAPHORGX = GRAPHORGX+50
Next (BINGRAPH)
Display Feature Histogram Results (#2, horizontal, differential, bins + graph (Y axis linear), statistics) Data Window (10, 871, 640, 300) Display Feature Histogram Results (#4, horizontal, differential, bins + graph (Y axis linear), statistics) Data Window (962, 880, 640, 300)
FILE BINDER AND MATERIAL HISTOGRAMS FOR CURRENT IMAGE
File Feature Histogram Results (#1, differential, statistics, bin details, channel #1)
File Line (channel #1)
File Feature Histogram Results (#3, differential, statistics, bin details, channel #2)
File Line (channel #2)
File Line (channel #2)
MEASURE MEAN SUBSTRATE THICKNESS
MFLDIMAGE = 4
Measure field (plane MFLDIMAGE, into FLDRESULTS(1), statistics into FLDSTATS(7,1))
   Selected parameters: Area
MEANTHICK = FLDRESULTS(1)/(CALVALUE*1330)
File ("Mean Substrate Thickness (um) = ", channel #1)
File (MEANTHICK, channel #1, 2 digits after'.')
File Line (channel #1)
File Line (channel #1)
Next (IMAGE)
FILE CUMMULATIVE BINDER AND MATERIAL HISTOGRAMS FOR CURRENT SLIDE
File Feature Histogram Results (#2, differential, statistics, bin details, channel #1)
File Feature Histogram Results (#4, differential, statistics, bin details, channel #2)
CLOSE DATA FILES
Close File (channel #1)
Close File (channel #2)
END

Prior to acquiring the first sample images, shading correction and color white balancing are performed using the QWIN software and a blank field-of-view illuminated only by the Leitz microscope. The system and images are also accurately calibrated using the QWIN software and a standard ruler with metric markings as small as one-tenth of a millimeter. The calibration is performed in the horizontal dimension of the video camera image.

After calibrating, the QUIPS algorithm Z-binder Distribution - 1 is executed via the QWIN system software and this initially prompts the analyst to place a single specimen slide onto the microscope auto-stage and within the field-of-view of the video camera. After positioning the specimen slide so the top material surface as observed when dispensing product is also the top surface as observed in the cross-sectional image, the specimen is properly aligned so that the horizontal middle of the section is in the field-of-view of the DFC 300 camera. The analyst will now be prompted to adjust the light level setting via the microscope power supply to register a white level reading of approximately 0.97. During this process of light adjustment, the QUIPS algorithm Z-binder Distribution - 1 will automatically display the current white level value within a small window on the video screen. If cross-sectioning artifacts are present in this center region of the cross-sectional image, the analyst should move the specimen slide horizontally to the immediate right or left (alternating direction between different cross-sections) until the next available 'clean' sampling region can be found.

After the light has been properly adjusted and the specimen positioned, via the auto-stage joystick, so that the cross-section is now also vertically centered in the field-of-view, the QUIPS algorithm Z-binder Distribution - 1 will then automatically acquire an image, perform image processing, electronically center the image, and make corresponding binder distribution in the z-direction measurements for a single cross-sectional specimen. The analyst will then be prompted to use the auto-stage joystick to reposition the specimen slide to the next adjacently cut cross-section, and focus appropriately, so that it can be imaged and analyzed accordingly. This repositioning step will occur two more times so that a third and forth specimen cross-section for the same subsample will be measured as well. The analysis of the four adjacently cut cross-sections constitute a single sub-sampling point of the material.

The binder distribution in the z-direction data are exported directly to an EXCEL® spreadsheet. Individual binder and total material z-distribution histograms are exported for data acquired from each image as well as a cumulative histogram for data from all four images. These latter cumulative histograms were used for calculating the percentage of binder in each one-third layer of the specimen thickness for a single material sub-sampling point. The area units are shown in the histogram are in square microns. In order to determine the histogram location of the top and bottom surface boundaries of the material, a 95 percent of total area rule was used on the total material histogram. In other words, when approaching the top and bottom material edges of the histogram, the surface boundary was considered to be the first histogram bin when a minimum of 2.5 percent material area had been encountered. These bin boundaries were then transposed over to the binder only cumulative histogram to determine the percentages of binder area present in the top, middle and bottom one-third histogram bins, inclusive of the calculated boundary bins. In cases where the number of bins was not evenly divisible by three (e.g. 8, 10, 14, etc.), a rotation technique was used to calculate binder percentages in each one-third layer of the material. For example, in the first encounter of a fourteen bin thickness, the top layer was four bins, the middle five, and the bottom five. During the next encounter, the top layer was five bins, the middle four, and the bottom five. If a third encounter occurs, the bottom layer would have one less or one more bin than the top and middle. If a fourth encounter occurs, the top layer again becomes the one containing one less or one more bin than the other two layers. This rotating method continues as required by the data.

The final sample mean binder percentage values for each one-third layer of z-distribution depth is based on an N=7 analysis from seven, separate, subsample regions each possessing four adjacently cut cross-sections. A comparison between different samples can be performed using a Student's T analysis at the 90 percent confidence level.

### EXAMPLES

### Example 1

The basesheet is made using an uncreped through-air-dried tissuemaking process in which a headbox deposits an aqueous suspension of papermaking fibers between forming wires. The newly-formed web is transferred from the forming wire to a slower moving transfer fabric with the aid of a vacuum box. The web is then transferred to a throughdrying fabric and passed over throughdryers to dry the web. After drying, the web is transferred from the throughdrying fabric to a reel fabric and thereafter briefly sandwiched between fabrics. The dried web remains with fabric until it is wound up into a parent roll.

To form the tissue, a headbox was employed, through which the 100percent softwood fibers and broke are pumped in a single layer. The fiber was diluted to between 0.19 and 0.29 percent consistency in the headbox to ensure uniform formation. The resulting single-layered sheet structure was formed on a twin-wire, suction form roll. The speed of the forming fabric was 1 km per minute (3304 feet per minute (fpm)). The newly-formed web was then dewatered to a consistency of about 20 to 27 percent using vacuum suction from below the forming fabric before being transferred to the transfer fabric, which was traveling at 0.85 cm per minute (2800 fpm) (18 percent rush transfer). A vacuum shoe pulling about 22.86 to 25.4 cm (9 to 10 inches) of mercury vacuum was used to transfer the web to the transfer fabric. A second vacuum shoe pulling about 12.7 to 15.24 cm (5 to 6 inches) of mercury vacuum was used to transfer the web to a t1207-12 throughdrying fabric manufactured by Voith Fabrics Inc. The web was carried over a pair of Honeycomb throughdryers operating at temperatures of about 191°C (375°F). and dried to a final dryness of about 97 to 99 percent consistency. The dried cellulosic web was rolled onto a core to form a parent roll of tissue.

A series of Unijet® nozzles, Nozzle type800050, manufactured by Spraying Systems Co., Wheaton, IL, operating at approximately 482 to 827 KPa (70 to 120 psi) were used to spray the binder composition onto both sides of the fibrous material. Each binder composition was sprayed at approximately 15 percent binder solids with water as the carrier. The wet partially formed single-ply wipe substrate was carried through a dryer operating at 177 to 204°C (350 to 400°F) at a speed of 107 metres per minute (350 fpm) to partially dry thesingle-ply wipe substrate. The partially dry wipe substrate was then wound on a core and then unwound and run through the 177 to 204°C (350 to 400°F) dryer a second time at a speed between 91.4 and 198 metres per minute (300 and 650 fpm) to raise the temperature of the wipe substrate to 121 to 177°C (250 to 350°F). The total dry weight percent of binder add-on was 5 percent relative to the dry mass of the single-ply wipe substrate. The basesheet was machine-converted into sections of continuous web 13.97 cm (5.5 inches) wide by 1.42m (56 inches) long with perforations every 17.78 cm (7 inches) which were adhesively joined, fan-folded and applied with the wetting composition at 235 percent add-on to yield a fan-folded stack of wet wipes. A wetting composition that is used on commercially available wet wipes under the trade designation KLEENEX® COTTONELLE FRESH® Folded Wipes (Kimberly-Clark Corporation of Neenah, WI) with the addition of 2 weight percent sodium chloride in the converting process for Example A. A wetting composition that is used on commercially available wet wipes under the trade designation KLEENEX® COTTONELLE FRESH® Folded Wipes (Kimberly-Clark Corporation of Neenah, WI) with the addition of 2 weight percent sodium chloride and 2 percent organopolysiloxane in the converting process for Example B.

The exemplary dispersible wipes were tested under the Shake Flask Test with each sample was tested at screen sizes of 12.70, 6.35, 3.18 and 1.59 mm with mass measured after the wipes and tensile strength test and compared to KLEENEX® COTTONELLE FRESH® Flushable Moist Wipes and Natural Choice@ Flushable Moist Wipes. Illustrative results are set forth below in Table 1.

**Table 1**

| **Example** | **Basesheet** | **In-Use Tensile Strength (MD) (g/line ar inch)** | **In-Use Tensile Strength (CD) (g/linear inch)** | **Shake Flask (% Mass Loss)** | | | |
|---|---|---|---|---|---|---|---|
| | | | | **12.70 mm** | **6.35 mm** | **3.18 mm** | **1.59 mm** |
| A | UCTAD | 568 | 272 | 100 | 100 | 98 | 63 |
| B | UCTAD | 513 | 264 | 100 | 100 | 97 | 51 |
| Comparative A | KLEENEX® COTTONELL EFRESH® | 374 | 271 | 100 | 85 | 63 | 28 |
| Comparative B | Natural Choice® | 560 | 205 | 20 | 20 | 20 | 10 |

As can be seen from these results, use of a basesheet made with cellulose fibers of length less than 3.18 mm have the necessary strength for use by consumers, but also more easily pass through smaller sieves. The exemplary wipes had percentage mass loss values of greater than 95 percent through 3.18 mm sieves, while the comparative examples do not. Thus, forming the single-ply wipe substrate from fibers shorter than those currently employed in commercially available wet wipes improves the Shaker Flash Test percent pass through. Accordingly, exemplary wet wipes A and B are likely to demonstrate improved flushability over currently commercially available wet wipes.

### Example 2

Examples A and B were tested to show the change in strength of the single-ply wipe from in-use to after-use when the wipe has split into two sections. To determine the after-use strength, samples of the wipes were agitated in tap water using the Slosh Box Test described above for a time period of ten minutes. After ten minutes, the samples have split into two sections through the central portion of the wipe. The After-Use Tensile strength was then measured. Illustrative results are set forth below in Table 2.

**Table 2**

| **Example** | **Tissue** | **In-Use Tensile Strength (MD) (g/linear inch)** | **In-Use Tensile Strength (CD) (g/linear inch)** | **After-Use Tensile Strength (MD) (g/linear inch)** | **After-Use Tensile Strength (CD) (g/linear inch)** |
|---|---|---|---|---|---|
| A | UCTAD | 568 | 272 | 133.7 | 71.9 |
| B | UCTAD | 513 | 264 | 126.2 | 74.1 |

As can be seen in Table 2, there is a significant loss in strength by splitting into two sections after only ten minutes in the Slosh Box. The loss of strength during the ply separation helps the product easily break up in waste water treatment process quickly to help prevent problems in the dispersibility of the wipes.

The samples were also tested using the Dutch Pump Test where a wipe is entered into a sewage pump and the pump's motor power was tracked to determine its effectiveness of handling the wipe. The test was done using wipes with the single ply individually separated into two sections after being submerged in water for ten minutes. The individual section was placed into the pump and showed a negligible power increase. To contrast, the samples were placed into the pump prior to separation. The power increase with the whole wipe was between 4 to 5 percent. This illustrates that ply separation provides better flushability than previous wipes that do not break apart into two sections.

### Example 3

For comparative purposes, a basesheet of airlaid nonwoven web was formed continuously on a commercial scale airlaid machine similar to the pilot-scale machine. Weyerhaeuser CF405 bleached softwood kraft fiber in pulp sheet form was used as the fibrous material. This airlaid fibrous material was densified to the desired level by heated compaction rolls and transferred to an oven wire, where it was sprayed on the top side with the a binder composition of a cationic polyacrylate that is the polymerization product of 96 mol% methyl acrylate and 4 mol% [2-(acryloyloxy)ethyl]trimethyl ammonium chloride and Airflex® EZ123 in a 70:30 ratio was used to bond the substrate binder composition, applying approximately half of the desired binder solids onto the dry fibrous material to prepare Comparative Example C. The airlaid basesheet is commonly used with KLEENEX® COTTONELLE FRESH® Flushable Moist Wipes.

Examples A and B and Comparative Example C were tested to show the air permeability of the basesheet. Illustrative results are set forth below in Table 3.

**Table 3**

| **Example** | **Basesheet** | **Air Permeability (cfm)** |
|---|---|---|
| A | UCTAD | 23.15 |
| B | UCTAD | 25.4 |
| Comparative C | Airlaid | 142 |

As can be seen in Table 3, there is a significant difference in the air permeability of the basesheet between uncreped through air-dried tissue and airlaid. A less air permeable sheet allows less of the binder into the middle sections of the basesheet. Thus, the binder is primarily on the outer surface portions of the basesheet made from uncreped through air-dried tissue. This will allow the single-ply substrate to delaminate into two sections and enhance the dispersibility of the wipe.

### Example 4

Example A and Comparative Example C were tested to show the binder distribution of the basesheet.

Illustrative results are set forth below in Table 4.

**Table 4**

| **Example** | **Basesheet** | **Binder Distribution in Top 1/3 Layer (%)** | **Binder Distribution in Middle 1/3 Layer (%)** | **Binder Distribution in Bottom 1/3 Layer (%)** |
|---|---|---|---|---|
| A | UCTAD | 39.7 | 13.5 | 46.8 |
| Comparative C | Airlaid | 45.3 | 28.7 | 26.0 |

As can be seen in Table 4, the binder distribution is substantially on the outer surfaces of the fibrous substrates of the uncreped through air-dried tissue, having only 13.5 percent of the binder within the middle layer. This is accomplished by using little or no vacuum to draw the spray though the sheet, using a relatively less permeable sheet to minimize airflow through the sheet, and using small amounts of binder composition.

Other modifications and variations to the appended claims may be practiced by those of ordinary skill in the art, without departing from the spirit and scope as set forth in the appended claims. It is understood that features of the various examples may be interchanged in whole or part. The preceding description, given by way of example in order to enable one of ordinary skill in the art to practice the claimed invention, is not to be construed as limiting the scope of the invention, which is defined by the claims and all equivalents thereto.

## Claims

1. A dispersible wet wipe comprising:
a single-ply wipe substrate comprising a fibrous substrate the fibrous substrate having outer surfaces and a central region, the fibrous substrate having a basis weight between 60 and 90 grams per square meter, and a binder composition including a triggerable polymer applied to outer surfaces of the fibrous substrate, and
a wetting composition containing between about 0.5 and about 3.5 percent of an insolubilizing agent; **characterised in that**
said fibrous substrate has an air permeability of between 0.283 and 2.83 m³ per minute (10 and 100 cubic feet per minute), and the majority of the binder composition is provided towards the outer surfaces of the fibrous substrate.

2. The dispersible wet wipe of claim 1 wherein said dispersible wet wipe splits into two sections upon agitation with water within a period of ten minutes or less.

3. The dispersible wet wipe of claim 1 wherein said binder composition is present at an add-on rate of between about 1 and about 15 percent.

4. The dispersible wet wipe of claim 1 wherein said binder composition is present at an add-on rate of between about 1 and about 8 percent.

5. The dispersible wet wipe of claim 1 wherein at least about 75 percent of the binder composition is distributed within one third of the outer surfaces of the fibrous substrate in a z-direction.

6. The dispersible wet wipe of claim 1 wherein the fibrous substrate comprises an uncreped through-air dried tissue web.

7. The dispersible wet wipe of claim 1 wherein the wet wipe has an in-use machine direction tensile strength of greater than 118 grams per linear cm (300 grams per linear inch).

8. The dispersible wet wipe of claim 2 wherein the two sections of the wet wipe has an after-use machine direction tensile strength of less than 78.7 grams per linear cm (200 grams per linear inch).

9. The dispersible wet wipe of claim 1 wherein said dispersible wet wipe splits into two sections upon contact with agitated tap water for a period of ten minutes.

10. The dispersible wet wipe of claim 1 wherein the fibrous substrate comprises a tissue web.

11. The dispersible wet wipe of claim 1 wherein said dispersible wet wipe has a pass through percentage value of at least about 70 percent.

12. The dispersible wet wipe of claim 8 wherein said dispersible wet wipe has a pass through percentage value of at least about 95 percent.

## Patentansprüche

1. Wegwerfbares Feuchttuch, umfassend:
ein einlagiges Tuchsubstrat, das ein Fasersubstrat umfasst, wobei das Fasersubstrat Außenflächen und einen Mittelbereich hat, das Fasersubstrat ein Flächengewicht zwischen 60 und 90 Gramm pro Quadratmeter hat und eine Bindemittelzusammensetzung, die ein triggerbares Polymer enthält, das auf die Außenflächen des Fasersubstrats aufgetragen ist, und
eine Benetzungszusammensetzung, die zwischen ungefähr 0,5 und ungefähr 3,5 Prozent eines Unlöslichmachers beinhaltet; **dadurch gekennzeichnet, dass** das Fasersubstrat eine Luftdurchlässigkeit zwischen 0,283 und 2,83 m³ pro Minute (10 und 100 Kubikfuß pro Minute) hat und der Großteil der Bindemittelzusammensetzung in Richtung der Außenflächen des Fasersubstrats bereitgestellt ist.

2. Wegwerfbares Feuchttuch nach Anspruch 1, wobei sich das wegwerfbare Feuchttuch bei Bewegung mit Wasser innerhalb eines Zeitraums von zehn Minuten oder weniger in zwei Abschnitte teilt.

3. Wegwerfbares Feuchttuch nach Anspruch 1, wobei die Bindemittelzusammensetzung bei einer Zugaberate zwischen ungefähr 1 und ungefähr 15 Prozent vorliegt.

4. Wegwerfbares Feuchttuch nach Anspruch 1, wobei die Bindemittelzusammensetzung bei einer Zugaberate zwischen ungefähr 1 und ungefähr 8 Prozent vorliegt.

5. Wegwerfbares Feuchttuch nach Anspruch 1, wobei zumindest ungefähr 75 Prozent der Bindemittelzusammensetzung innerhalb eines Drittels der Außenflächen des Fasersubstrats in einer z-Richtung verteilt sind.

6. Wegwerfbares Feuchttuch nach Anspruch 1, wobei das Fasersubstrat eine nicht gekreppte Gewebebahn umfasst, die durchluftgetrocknet ist.

7. Wegwerfbares Feuchttuch nach Anspruch 1, wobei das Feuchttuch eine Reißfestigkeit in Verwendung in Maschinenrichtung von mehr als 118 Gramm pro linearem cm (300 Gramm pro linearem Inch) hat.

8. Wegwerfbares Feuchttuch nach Anspruch 2 wobei die zwei Abschnitte des feuchten Wischtuchs nach Verwendung eine Reißfestigkeit in Maschinenrichtung von weniger als 78,7 Gramm pro linearem cm (200 Gramm pro linearem Inch) haben.

9. Wegwerfbares Feuchttuch nach Anspruch 1, wobei sich das wegwerfbare Feuchttuch bei Kontakt mit bewegtem Leitungswasser für einen Zeitraum von zehn Minuten in zwei Abschnitte teilt.

10. Wegwerfbares Feuchttuch nach Anspruch 1, wobei das Fasersubstrat eine Gewebebahn umfasst.

11. Wegwerfbares Feuchttuch nach Anspruch 1, wobei das wegwerfbare Feuchttuch einen Durchlaufprozentwert von zumindest ungefähr 70 Prozent hat.

12. Wegwerfbares Feuchttuch nach Anspruch 8, wobei das wegwerfbare Feuchttuch einen Durchlaufprozentwert von zumindest ungefähr 95 Prozent hat.

## Revendications

1. Serviette humide dispersible comprenant :
un substrat de serviette à pli unique comprenant un substrat fibreux, le substrat fibreux ayant des surfaces extérieures et une région centrale, le substrat fibreux ayant un poids de base entre 60 et 90 grammes par mètre carré, et une composition de liant comprenant un polymère déclenchable appliqué à des surfaces extérieures du substrat fibreux, et
une composition humidifiante contenant entre environ 0,5 et environ 3,5 pour cent d'un agent insolubilisant ; **caractérisée en ce que** ledit substrat fibreux a une perméabilité à l'air entre 0,283 et 2,83 m³ par minute (10 et 100 pieds cubiques par minute), et la majorité de la composition de liant est fournie vers les surfaces extérieures du substrat fibreux.

2. Serviette humide dispersible selon la revendication 1, dans laquelle ladite serviette humide dispersible se divise en deux sections lors de l'agitation avec de l'eau en dix minutes ou moins.

3. Serviette humide dispersible selon la revendication 1, dans laquelle ladite composition de liant est présente à un taux d'apport entre environ 1 et environ 15 pour cent.

4. Serviette humide dispersible selon la revendication 1, dans laquelle ladite composition de liant est présente à un taux d'apport entre environ 1 et environ 8 pour cent.

5. Serviette humide dispersible selon la revendication 1, dans laquelle au moins 75 pour cent de la composition de liant est distribuée dans un tiers des surfaces extérieures du substrat fibreux dans une direction z.

6. Serviette humide dispersible selon la revendication 1, dans laquelle le substrat fibreux comprend une bande de tissu séchée à l'air non crêpée.

7. Serviette humide dispersible selon la revendication 1, dans laquelle la serviette humide a une résistance à la traction en direction de la machine en utilisation supérieure à 118 grammes par cm linéaire (300 grammes par pouce linéaire).

8. Serviette humide dispersible selon la revendication 2, dans laquelle les deux sections de la serviette humide ont une résistance à la traction en direction de la machine après utilisation inférieure à 78,7 grammes par cm linéaire (200 grammes par pouce linéaire).

9. Serviette humide dispersible selon la revendication 1, dans laquelle ladite serviette humide dispersible se divise en deux sections lors du contact avec de l'eau du robinet agitée pendant dix minutes.

10. Serviette humide dispersible selon la revendication 1, dans laquelle le substrat fibreux comprend une bande de tissu.

11. Serviette humide dispersible selon la revendication 1, dans laquelle ladite serviette humide dispersible a une valeur de pourcentage de traversée d'au moins environ 70 pour cent.

12. Serviette humide dispersible selon la revendication 8, dans laquelle ladite serviette humide dispersible a une valeur de pourcentage de traversée d'au moins environ 95 pour cent.
